# EUROPEAN PATENT SPECIFICATION

(11) **EP 1 969 169 B1**
(45) Date of publication and mention of the grant of the patent: **02.11.2011**
(21) Application number: 06815677.7
(22) Date of filing: 28.09.2006
(51) Int. Cl.: D04H 1/64, A61L 15/62

(54) **DISPERSIBLE WET WIPES WITH IMPROVED DISPENSING**
DISPERGIERBARE FEUCHTWISCHTÜCHER MIT VEBESSERTER ABGABE
LINGETTES HUMIDES DISPERSIBLES DE DISTRIBUTION AMELIOREE

(30) Priority: 15.12.2005 US 300967
(43) Date of publication of application: 17.09.2008
(73) Proprietor: KIMBERLY-CLARK WORLDWIDE, INC., Neenah, WI 54956 (US)
(72) Inventor: BUNYARD, William, Clayton, De Pere, Wisconsin 54115 (US); BRANHAM, Kelly, Dean, Woodstock, Georgia 30188 (US); LOSTOCCO, Michael, Ralph, Appleton, Wisconsin 54913 (US); DYER, Thomas, Joseph, Neenah, Wisconsin 54956 (US); HOCKERSMITH, Jeffrey, Michael, Mill Creek, Washington 98012 (US); POSSELL, Kevin, Christopher, Middleton, Wisconsin 53562 (US)
(74) Representative: Zimmermann & Partner
(86) International application number: PCT/US2006/037847
(87) International publication number: WO 2007/070147

(56) References cited:
- EP-A1- 0 857 453
- WO-A-00/57843
- WO-A-02/077042
- WO-A-2004/026351
- US-A1- 5 540 332

## Description

### BACKGROUND

For many years, the problem of disposability has plagued industries that provide disposable items, such as diapers, wet wipes, incontinence garments and feminine care products. Ideally, when a disposable product intended to be discarded in either sewer or septic systems, the product, or designated portions of the product, should "disperse" and thus sufficiently dissolve or disintegrate in water so as not to present problems under conditions typically found in either household and municipal sanitization systems. While much headway has been made in addressing this problem, one of the weak links has been the inability to create economical nonwoven materials, which are readily dispersible but still have sufficient in-use properties such as strength, thickness, opacity, absorbency, softness, flexibility, cleansing, ease-of-use, etc. to make consumer acceptable products. These nonwoven materials may be formed by wet or dry (air) layering of a generally random plurality of fibers that are joined together adhesively and/or physically. Under appropriate conditions, nonwoven materials will dissolve or disintegrate in water such as by simple dilution in an excess of water or dilution in excess water with the application of appropriate shear force.

U.S. Pat. Application Nos. 10/830,558, 10/251,610, and 9/900,698 illustrates a number of approaches to adhesively-bonded dispersible nonwoven webs and U.S. Pat. No. 4,755,421 illustrates a variety of approaches to physically-bonded dispersible nonwoven fabrics, such as those formed via hydroentangling methods. WO 2004/026351 discloses fiber-containing fabrics and webs comprising ion-triggerable, water-dispersible binder compositions and their applicability in water-dispersible personal care products, such as wet wipes.

Typically, wet wipes are stacked in a container in either a folded or unfolded configuration. For example, containers of wet wipes are available wherein each of the wet wipes are arranged in a folded configuration, such as c-folded, z-folded, or quarter-folded configurations as are well known to those skilled in the art. Sometimes the folded wet wipes are also interfolded with the wet wipes immediately above and below in the stack of wet wipes. In yet other configurations, the wet wipes are placed in the container in the form of a continuous nonwoven material. In this case, each individual wet wipe or sheet may be connected, from the first sheet to the last, by similarly weakened lines of perforations or by adhesive bonds. These wet wipes can be stacked on top of each other in a fan folded manner or can be wound into a roll configuration.

Unfortunately, when dispersible nonwoven materials have been employed as wet wipes, dispensing of the wet wipes has not been completely satisfactory. Unsatisfactory dispensing has been encountered, particularly in the case of wet wipes that are formed from adhesively-bonded dispersible nonwoven materials. Poor dispensing can be ascribed to a variety of factors, one of which is sheet-to-sheet adhesion, which is addressed herein.

Sheet-to-sheet adhesion is the tendency of a wet wipe to adhere to itself or adjacent wet wipes. Sheet-to-sheet adhesion can be attributed to a number of factors, some of which include compression of stacked or rolled wet wipes during manufacturing, attractive interactions between the nonwoven material and a wetting composition, and interactions between the surfaces of adjacent, contacting nonwoven wet wipe. If the sheet-to-sheet adhesion is sufficiently high, single-handed, one-at-a-time dispensing of the wet wipes can be problematic. This problem can be particularly acute when the individual wet wipes in the stack are folded such that the leading edge of each wet wipe is folded over another portion of the wet wipe. If the leading end edge of the wet wipe has a high affinity for the wet wipe underlying it (high sheet-to-sheet adhesion), it can be undesirably difficult for the user to identify and grasp the leading end edge and peelingly lift it from the underlying stack of wet wipes. If the sheet-to-sheet adhesion is sufficiently high, tearing of the wet wipe can occur when attempting to remove the leading wet wipe from the top of the wet wipe stack.

Moreover, when the user removes an individually folded wet wipe from the underlying stack, high sheet-to-sheet adhesion can result in undesirable incomplete unfolding of the wet wipe. Additionally, high sheet-to-sheet adhesion can cause an individual folded wet wipe to remain partially adhered to the adjacent wet wipe, upon which it is resting, thus causing dispensing of multiple wet wipes rather than the preferred dispensing of an individual wet wipe. Such difficulties in separation and incomplete unfolding have undesirably resulted in reduced consumer acceptance.

This sheet-to-sheet adhesion issue has been addressed in the prior art for non-dispersible wet wipes. For example, US Pat. 5,540,332 describes a wet wipe with improved dispensability, wherein the leading edge of the wet wipe utilizes a repeating non-linear pattern (such as a sinusoidal pattern) to facilitate a reduced peel force for dispensing. In another example, EP 0 857,453 B1 describes a folded wet wipe with improved dispensability, wherein the coefficient of friction is reduced on the wet wipe surfaces through embossing or chemical means.

Unfortunately, these approaches to addressing the dispensing problems caused by sheet-to-sheet adhesion are not sufficient to counteract the considerably higher sheet-to-sheet adhesion that can be observed when the wet wipe is a dispersible nonwoven material, particularly when the wet wipe is in a stacked and/or folded configuration.

### BRIEF SUMMARY

In one aspect of the invention, there is a wet wipe that includes a nonwoven web and an aqueous wetting composition. The nonwoven web includes a fibrous material and a binder composition. The binder composition includes a triggerable polymer and an anti-blocking agent, as set out in claim 1 wherein the binder composition is insoluble in the wetting composition. The wet wipe, as discussed in this aspect, is dispersible in water having a total dissolved solids up to about 500 ppm and a CaCO₃ equivalent hardness up to about 250 ppm.

In a further aspect of the invention, there is a wet wipe that includes a nonwoven web and an aqueous wetting composition. The nonwoven web includes a fibrous material, an anti-blocking coating, as set out in claim 1 and a binder composition. The binder composition includes a triggerable polymer and is insoluble in the wetting composition. The wet wipe, as described in this aspect, is dispersible in water having a total dissolved solids up to about 500 ppm and a CaCO₃ equivalent hardness up to about 250 ppm.

### DETAILED DESCRIPTION

A wet wipe possessing improved dispensing, is disclosed herein, wherein the wet wipemay preferably be dispersible. In one embodiment, the wet wipemay desirably be adhesively bonded with a triggerable polymer. The term "triggerable" refers to the ability of this polymer to selectively provide the wet wipe with the desired in-use strength, while also providing it with the ability to lose sufficient strength such that the wet wipe will disperse when disposed in tap water, such as is found in toilets for example.

The wet wipe comprises a nonwoven material that is wetted with an aqueous solution termed the "wetting composition". The nonwoven material may comprise either a nonwoven fabric or a nonwoven web. The nonwoven fabric may comprise a fibrous material, while the nonwoven web may comprise the fibrous material and a binder composition. In another embodiment, the nonwoven material may also comprise an anti-blocking coating. The binder composition may comprise a triggerable polymer and either an anti-blocking agent or a cobinder. Although the anti-blocking agent and the anti-blocking coating may be selected from the same group of materials, as will be discussed in more detail below, the anti-blocking agent and the anti-blocking coating may be distinguished based on how and when they are applied during formation of the wet wipe. The anti-blocking agent may preferably be applied to the fibrous material as a component of the binder composition, while the anti-blocking coating may preferably be applied to the surface of the nonwoven material.

The wetting composition desirably maintains the insolubility of the binder composition and may comprise an aqueous composition containing an insolubilizing agent. When the wet wipe is exposed to tap water, the wetting composition dilutes and the binder composition desirably loses strength leading to concomitant fragmentation and dispersal of the wet wipe. Thus, the combination of the binder composition and the wetting composition preferably afford the structural integrity or coherency necessary to maintain the in-use strength and properties of the wet wipe, while also allowing for selective fragmentation or dispersal of the wet wipe under desired conditions.

In one embodiment, the nonwoven web of the wet wipe may be generated by spraying the fibrous material with the binder composition, wherein the binder composition comprises a mixture or solution comprising both the triggerable polymer and the anti-blocking agent. In a further embodiment, the binder composition may comprise the triggerable polymer, but no antiblocking agent. In another embodiment, the nonwoven web of the wet wipe may be generated by spraying the fibrous material with the binder composition, wherein the binder composition comprises the triggerable polymer and the anti-blocking agent such that these components of the binder composition are applied sequentially. For example, the triggerable polymer may be applied first, followed by the anti-blocking agent. In an alternative embodiment, the anti-blocking agent may be applied first, followed by the triggerable polymer. In an additional embodiment, the nonwoven material of the wet wipe may be generated by applying the anti-blocking coating to the surface of the nonwoven material, where the nonwoven material in this embodiment comprises the fibrous material, the triggerable polymer and either the anti-blocking agent or the cobinder. In a further embodiment, the nonwoven material of the wet wipe may be generated by applying the anti-blocking coating to the surface of the nonwoven material, where the nonwoven material in this embodiment contains the triggerable polymer, without antiblocking agent and/or cobinder.

### Nonwoven Material

In many personal care products, nonwoven materials are the preferred substrate, especially with regard to wet wipes. As previously discussed, two types of nonwoven materials are described herein, the "nonwoven fabrics" and the "nonwoven webs". As used herein, the nonwoven fabric comprises a fibrous material or substrate, where the fibrous material or substrate comprises a sheet that has a structure of individual fibers or filaments randomly arranged in a mat-like fashion and does not include the binder composition. Nonwoven fabrics may be made from a variety of processes including, but not limited to, airlaid processes, wet-laid processes such as with cellulosic-based tissues or towels, hydroentangling processes, staple fiber carding and bonding, and solution spinning.

Since nonwoven fabrics do not include a binder composition, the fibrous substrate used for forming the nonwoven fabric may desirably have a greater degree of cohesiveness and/or tensile strength than the fibrous substrate that is used for forming the nonwoven web. For this reason nonwoven fabrics comprising fibrous subtrates created via hydroentangling may be particularly preferred for formation of the nonwoven fabric. Hydroentangled fibrous materials may provide the desired in-use strength properties for wet wipes that comprise a nonwoven fabric.

With regard to the nonwoven web, the binder composition may be applied to the fibrous material or substrate to form the nonwoven web using a variety of techniques.. The fibrous material used to form the nonwoven web, may desirably have a relatively low wet cohesive strength prior to its treatment with the binder composition. Thus, when the fibrous substrate is bonded together by the binder composition, the nonwoven web will preferably break apart when it is placed tap water, such as found in toilets and sinks. Thus the identity of the fibrous material may depend on whether it is to be used to form the nonwoven fabric or the nonwoven web. Furthermore, the fibers from which the fibrous material is made may also be selected based on whether they are to be used for a nonwoven web or nonwoven fabric.

The fibers forming the fibrous material may be made from a variety of materials including natural fibers, synthetic fibers, and combinations thereof. The choice of fibers may depend upon, for example, the intended end use of the finished substrate, the fiber cost and whether fibers will be used for a nowoven fabric or a nonwoven web. For instance, suitable fibers may include, but are not limited to, natural fibers such as cotton, linen, jute, hemp, wool, wood pulp, etc. Similarly, suitable fibers may also include: regenerated cellulosic fibers, such as viscose rayon and cuprammonium rayon; modified cellulosic fibers, such as cellulose acetate; or synthetic fibers, such as those derived from polypropylenes, polyethylenes, polyolefins, polyesters, polyamides, polyacrylics, etc.. Regenerated cellulose fibers, as briefly discussed above, include rayon in all its varieties as well as other fibers derived from viscose or chemically modified cellulose, including regenerated cellulose and solvent-spun cellulose, such as Lyocell. Among wood pulp fibers, any known papermaking fibers may be used, including softwood and hardwood fibers. Fibers, for example, may be chemically pulped or mechanically pulped, bleached or unbleached, virgin or recycled, high yield or low yield, and the like. Chemically treated natural cellulosic fibers may be used, such as mercerized pulps, chemically stiffened or crosslinked fibers, or sulfonated fibers.

In addition, cellulose produced by microbes and other cellulosic derivatives may be used. As used herein, the term "cellulosic" is meant to include any material having cellulose as a major constituent, and, specifically, comprising at least 50 percent by weight cellulose or a cellulose derivative. Thus, the term includes cotton, typical wood pulps, non-woody cellulosic fibers, cellulose acetate, cellulose triacetate, rayon, thermomechanical wood pulp, chemical wood pulp, debonded chemical wood pulp, milkweed, or bacterial cellulose. Blends of one or more of any of the previously described fibers may also be used, if so desired.

The fibrous material may be formed from a single layer or multiple layers. In the case of multiple layers, the layers are generally positioned in a juxtaposed or surface-to-surface relationship and all or a portion of the layers may be bound to adjacent layers. The fibrous material may also be formed from a plurality of separate fibrous materials wherein each of the separate fibrous materials may be formed from a different type of fiber. In those instances where the fibrous material includes multiple layers, the binder composition may be applied to the entire thickness of the fibrous material, or each individual layer may be separately treated and then combined with other layers in a juxtaposed relationship to form the finished fibrous material.

Airlaid nonwoven fabrics are particularly well suited for use as wet wipes. The basis weights for airlaid nonwoven fabrics may range from about 20 to about 200 grams per square meter (gsm) with staple fibers having a denier of about 0.5-10 and a length of about 6-15 millimeters. Wet wipes may generally have a fiber density of about 0.025 g/cc to about 0.2 g/cc. Wet wipes may generally have a basis weight of about 20 gsm to about 150 gsm. More desirably the basis weight may be from about 30 to about 90 gsm. Even more desirably the basis weight may be from about 50 gsm to about 60 gsm.

### Binder Composition

In another embodiment, the binder composition may comprise the triggerable polymer, a cobinder polymer and/or an antiblocking agent. In addition to providing the wet wipe with in-use strength in the presence of the wetting composition and selective dispersibility in tap water, the binder composition preferably possesses a variety of other desirable properties. For example, the binder composition may preferably be processable on a commercial scale (i.e., the binder may be capable of rapid application on a large scale, such as by spraying) and may also desirably be inexpensive. The binder composition may also desirably provide acceptable levels of sheet wettability. In addition, all components of the wet wipe, including the binder composition, may preferably be non-toxic and relatively economical.

The amount of binder composition present in the nonwoven web may desirably range from about 5 to about 65 percent by weight based on the total weight of the nonwoven web. More desirably, the binder composition may comprise from about 7 to about 35 percent by weight based on the total weight of the nonwoven web. Even more desirably, the binder composition may comprise from about 10 to about 25 percent by weight based on the total weight of the nonwoven web. Most desirably, the binder composition may comprise from about 15 to 20 percent by weight based on the total weight of the nonwoven web. The amount of the binder composition desirably results in a nonwoven web that has in-use integrity, but quickly disperses when soaked in tap water.

The composition of tap water can vary greatly depending on the water source. Thus the binder composition may preferably be capable of loosing sufficient strength to allow the wet wipe to disperse in tap water covering the preponderance of the tap water composition range found throughout the United States (and throughout the world). Thus, it is important to evaluate the dispersibility of the binder composition in aqueous solutions which contain the major components in tap water and in a representative concentration range encompassing the majority of the tap water sources in the United States. The predominant inorganic ions typically found in drinking water are sodium, calcium, magnesium, bicarbonate, sulfate and chloride. Based on a recent study conducted by the American Water Works Association (AWWA) in 1996, the predominance of the U.S. municipal water systems (both ground water and surface water sources) surveyed have a total dissolved solids of inorganic components of about 500 ppm or less. This level of 500 ppm total dissolved solids also represents the secondary drinking water standard set by the U.S. Environmental Protection Agency. The average water hardness, which represents the calcium and magnesium concentrations in the tap water source, at this total dissolved solids level was ca. 250 ppm (CaCO₃ equiv.), which also encompasses the water hardness for the predominance of the municipal water systems surveyed by the AWWA. As defined by the United States Geological Survey (USGS), a water hardness of 250 ppm equiv. CaCO₃ would be considered "very hard" water. Similarly, the average bicarbonate concentration at 500 ppm total dissolved solids reported in the study was ca.112 ppm, which also encompasses the bicarbonate, or alkalinity, of the predominance of the municipal water systems surveyed. A past study by the USGS of the finished water supplies of 100 of the largest cities in the United States suggests that a sulfate level of about 100 ppm is sufficient to cover the majority of finished water supplies. Similarly, sodium and chloride levels of at least 50 ppm each should be sufficient to cover the majority of U.S. finished water supplies. Thus, binder compositions which are capable of loosing strength in tap water compositions meeting these minimum requirements should also lose strength in tap water compositions of lower total dissolved solids with varied compositions of calcium, magnesium, bicarbonate, sulfate, sodium, and chloride.

To ensure the dispersibility of the binder composition across the country (and throughout the whole world), the binder composition may desirably be soluble in water containing up to about 100 ppm total dissolved solids and a CaCO₃ equivalent hardness up to about 55 ppm. More desirably, the binder composition may be soluble in water containing up to about 300 ppm of total dissolved solids and a CaCO₃ equivalent hardness up to about 150 ppm. Even more desirably, the binder composition may be soluble in water containing up to about 500 ppm total dissolved solids and a CaCO₃ equivalent hardness up to about 250 ppm.

### Triggerable Polymer

As previously discussed, the binder composition may comprise the triggerable polymer, the anti-blocking agent and/or the cobinder. A variety of triggerable polymers may be used. One type of triggerable polymer is a dilution triggerable polymer. Examples of dilution triggerable polymers include ion-sensitive polymers, which may be employed in combination with a wetting composition in which the insolubilizing agent is a salt. Other dilution triggerable polymers may also be employed, wherein these dilution triggerable polymers are used in combination with wetting agents using a variety of insolubilizing agents, such as organic or polymeric compounds.

Although the triggerable polymer may be selected from a variety of polymers, including temperature sensitive polymers and pH-sensitive polymers, the triggerable polymermay preferably be the dilution triggerable polymer, comprising the ion-sensitive polymer. If the ion-sensitive polymer is derived from one or more monomers, where at least one contains an anionic functionality, the ion-sensitive polymer is referred to as an anionic ion-sensitive polymer. If the ion-sensitive polymer is derived from one or more monomers, where at least one contains a cationic functionality, the ion-sensitive polymer is referred to as a cationic ion-sensitive polymer. An exemplary anionic ion-sensitive polymer is described in U.S. Pat. No. 6,423,804.

Examples of cationic ion-sensitive polymers are disclosed in the following U.S. Patent Application Publication Nos.: 2003/0026963 A1; 2003/0027270 A1; 2003/0032352 A1; 2004/003008.0 A1; 2003/0055146 A1; 2003/0022568 A1; 2003/0045645 A1; 2004/0058600 A1; 2004/0058073 A1; 2004/0063888 A1; 2004/0055704 A1; 2004/0058606 A1; and 2004/0062791 A1,

Desirably, the ion-sensitive polymer may be insoluble in the wetting composition, wherein the wetting composition comprises at least about 0.3 weight percent of an insolubilizing agent which may be comprised of one or more inorganic and/or organic salts containing monovalent and/or divalent ions. More desirably, the ion-sensitive polymer may be insoluble in the wetting composition, wherein the wetting composition comprises from about 0.3% to about 10% by weight of an insolubilizing agent which may be comprised of one or more inorganic and/or organic salts containing monovalent and/or divalent ions. Even more desirably, the ion-sensitive polymer may be insoluble in the wetting composition, wherein the wetting comoposition comprises from about 0.5% to about 5% by weight of an insolubilizing agent which comprises one or more inorganic and/or organic salts containing monovalent and/or divalent ions. Especially desirably, the ion-sensitive polymer may be insoluble in the wetting composition, wherein the wetting composition comprises from about 1.0% to about 4.0% by weight of an insolubilizing agent which comprises one or more inorganic and/or organic salts containing monovalent and/or divalent ions. Suitable monovalent ions include, but are not limited to, Na⁺ ions, K⁺ ions, Li⁺ ions, NH₄⁺ ions, low molecular weight quaternary ammonium compounds (e.g., those having fewer than 5 carbons on any side group), and a combination thereof. Suitable divalent ions include, but are not limited to, Zn²⁺, Ca²⁺ and Mg²⁺. These monovalent and divalent ions may be derived from organic and inorganic salts including, but not limited to, NaCl, NaBr, KCl, NH₄Cl, Na₂SO₄, ZnCl₂, CaCl₂, MgCl₂, MgSO₄, and combinations thereof. Typically, alkali metal halides are the most desirable monovalent or divalent ions because of cost, purity, low toxicity, and availability. A particularly desirable salt is NaCl.

In a preferred embodiment, the ion-sensitive polymer may desirably provide the nonwoven web with sufficient in-use strength (typically >300 g/in.) in combination with the wetting composition containing sodium chloride. These nonwoven webs may be dispersible in tap water (including water with), desirably losing most of their wet strength (<100 g/in.) in 24 hours, or less.

In another preferred embodiment, the ion-sensitive polymer may comprise the cationic sensitive polymer, wherein the cationic sensitive polymer is a cationic polyacrylate that is the polymerization product of 96 mol% methyl acrylate and 4 mol% [2-(acryloyloxy)ethyl]trimethyl ammonium chloride.

### Cobinder Polymers

As previously discussed, the binder composition may comprise the triggerable polymer, the anti-blocking agent and/or the cobinder. When the binder composition comprises the triggerable polymer and the cobinder, the triggerable polymer and the cobinder may preferably be compatible with each other in aqueous solutions to: 1) allow for facile application of the binder composition to the fibrous substrate in a continuous process and 2) prevent interference with the dispersibility of the binder composition. Therefore, if the triggerable polymer is the anionic ion-sensitive polymer, cobinders which are anionic, nonionic, or very weakly cationic may be preferred. If the triggerable polymer is the cationic ion-sensitive polymer, cobinders which are cationic, nonionic, or very weakly anionic may be. Additionally, the cobinder desirably does not provide substantial cohesion to the nonwoven material by way of covalent bonds,such that it interferes with the dispersibility of the nonwoven web.

The presence of the cobinder may provide a number of desirable qualities. For example, the cobinder may serve to reduce the shear viscosity of the triggerable polymer, such that the binder composition has improved sprayability over the triggerable binder alone. By use of the term "sprayable" it is meant that these polymers may be applied to the fibrous material or substrate by spraying, allowing the uniform distribution of these polymers across the surface of the substrate and penetration of these polymers into the substrate. The cobinder may also reduce the stiffness of the nonwoven web compared to the stiffness of a nonwoven web to which only the triggerable polymer has been applied. Reduced stiffness may be achieved if the cobinder has a glass transition temperature, T_{g}, that is lower than the T_{g} of the triggerable polymer. In addition, the cobinder may be less expensive than the triggerable polymer and by reducing the amount of triggerable polymer needed, may serve to reduce the cost of the binder composition. Thus, it may be desirable to use the highest amount of cobinder possible in the binder composition such that it does not jeopardize the dispersibility and in-use strength properties of the wet wipe. In a preferred embodiment, the cobinder replaces a portion of the triggerable polymer in the binder composition and permits a given strength level to be achieved, relative to a wet wipe having approximately the same tensile strength but containing only the triggerable polymer in the binder composition, to provide at least one of the following attributes: lower stiffness; better tactile properties (e.g. lubricity or smoothness); or reduced cost.

In one embodiment, the cobinder present in the binder composition, relative to the mass of the binder composition, may be about 10% or less, more desirably about 15% or less, more desirably 20% or less, more desirably 30% or less, or more desirably about 45% or less. Exemplary ranges of cobinder relative to the solid mass of the binder composition may include from about 1% to about 45%, from about 25% to about 35%, from about 1% to about 20% and from about 5% to about 25%.

The cobinder may be selected from a wide variety of polymers, as are known in the art. For example, the cobinder may be selected from the group consisting of poly(ethylene--vinyl acetate), poly(styrene-butadiene), poly(styrene-acrylic), a vinyl acrylic terpolymer, a polyester latex, an acrylic emulsion latex, poly vinyl chloride, ethylene-vinyl chloride copolymer, a carboxylated vinyl acetate latex, and the like. A variety of additional exemplary cobinder polymers are discussed in U.S. Patent No. 6,653,406 and U.S. Patent Application Publication 2003/00326963.

### Anti-blocking Agents and Anti-blocking Coating Polymers

The anti-blocking agent and anti-blocking coating may be selected from a variety of similar polymeric materials. The anti-blocking agent and the anti-blocking coating are defined as polymeric materials that reduce or prevent the tendency of two adjacent layers of a material to stick together, particularly when under pressure or exposed to high ambient temperatures. In the case of wet wipes, the anti-blocking agent and the anti-blocking coating may desirably prevent the tendency of two adjacent sheets of wet wipe to adhere to one another, thereby reducing the sheet-to-sheet adhesion. Although the anti-blocking agent and the anti-blocking coating may be selected from similar polymeric materials, the anti-blocking agent and the anti-blocking coating may be distinguished based on how and when they are applied during formation of the wet wipe. The anti-blocking agent may preferably be applied to the fibrous substrate as a component of the binder composition, while the anti-blocking coating may preferably be applied to the surface of the nonwoven material, whether the nonwoven material is a nonwoven web or a nonwoven fabric.

The triggerable polymer and the anti-blocking agent may preferably be compatible with each other in aqueous solutions to allow for facile application of the binder composition to the fibrous material in a continuous process and to prevent interference with the dispersibility of the triggerable polymer. Therefore, if the triggerable polymer is an anionic ion-sensitive polymer, the anti-blocking agent may desirably be anionic, nonionic, or very weakly cationic. If the triggerable polymer is a cationic ion-sensitive polymer, the anti-blocking agent may desirably be cationic, nonionic, or very weakly anionic. In addition, the anti-blocking agent may desirably be of a type, and present in an amount, such that when combined with the triggerable polymer, the anti-blocking agent is compatible with the triggerable polymer, thus allowing a mixture of the triggerable polymer and the anti-blocking agent to be sprayable.

The anti-blocking agent may be present, relative to the mass of the total binder composition, in an amount of about 30% or less, desirably about 25% or less, more desirably about 20% or less, more desirably about 15% or less, and more desirably about 10% or less, with exemplary ranges of from about 1% to about 30% or from about 15% to about 25%, as well as from about 1% to about 15% or from about 5% to about 20%. The amount of anti-blocking agent present may desirably be low enough, such that the anti-blocking agent is present as a discontinuous phase. When the anti-blocking agent is present as a discontinuous phase, an insufficient number of insoluble regions of the anti-blocking agent may be present to negatively impact the dispersibility of the nonwoven material.

The anti-blocking coating may be selected from the same previously discussed polymeric materials as the anti-blocking agent, as long as the polymeric material can be applied to the surface of the nonwoven material in a continuous fashion by methods known in the art, such as printing, foaming or spraying, for example. As used herein, the anti-blocking coating refers to deposits or discontinuous regions of polymeric material preferentially located on the surface of the nonwoven material. Additionally, the anti-blocking coating may be selected from polymeric materials that are not compatible with the triggerable polymer in aqueous solution. For example in one embodiment, an anionic polymer dispersion, which is not compatible with a cationic ion-sensitive polymer in aqueous solution, may be used as an anti-blocking coating for a nonwoven web comprising the cationic ion-sensitive polymer. The anti-blocking coating may desirably be selected such that it is sufficiently compatible with the triggerable polymer as to not interfere with the dispersibility of the nonwoven web when applied to the surface of the nonwoven web.

The anti-blocking coating may be present at a level relative to the total nonwoven material of about 15% or less, desirably about 10% or less, more desirably about 7% or less, more desirably about 3% or less, and more desirably about 1% or less. The amount of anti-blocking coating present may desirably be low enough that the anti-blocking coating forms a plurality of discontinuous deposits on the nonwoven material surface and may desirably be unable to create enough insoluble bonded regions to jeopardize the dispersibility of the coated nonwoven web.

To achieve reduced sheet-to-sheet adhesion when utilized in dispersible wet wipes, the antiblocking agent and antiblocking coating may desirably have the physical properties discussed below. When the anti-blocking agent and the anti-blocking coating are an amorphous polymeric material, the T_{g} may be the characteristic of concern, while in the case of a semi-crystalline polymeric material, the melting temperature (Tₘ) may be the characteristic of primary concern. In order to mitigate sheet-to-sheet adhesion, the polymeric material, from which the anti-blocking agent and the anti-blocking coating are selected, may desirably possess a T_{g} (for amorphous materials) or Tₘ (for semi-crystalline materials) that is close to or greater than the storage temperature of the moist wet wipe. In a further embodiment, the polymeric material, from which the anti-blocking agent and the anti-blocking coating are selected, may desirably possess a T_{g} or Tₘ that is close to or greater than room temperature.

While not wishing to be bound by any theory, it is believed that when the triggerable polymer is applied to the fibrous material, such as by spraying, a plurality of binder domains on the surface of the nonwoven web are formed after drying, wherein these domains comprise a plurality of triggerable polymer molecules. The triggerable polymer molecules located on the surface of such domains may come into intimate contact with binder domains on the surface of an adjacent wet wipe surface (e.g., between two different wet wipes or between two portions of a folded wet wipe). If the triggerable polymer possesses a T_{g} sufficiently below ambient temperature, or is plasticized by the wetting composition such that the T_{g} of the triggerable polymer in the wet wipe is below ambient temperature, the molecules of triggerable polymer on adjacent wet wipe surfaces may have sufficient mobility to entangle and thus cohesively weld together the intimately contacting surfaces of the binder domains on adjacent wipe surfaces, resulting in sheet-to-sheet adhesion. Triggerable polymers, such as ion-sensitive polymers, possess a relatively high affinity for water, and may thus exhibit a depressed T_{g} in the wet wipe versus the dry state due to plasticization by the wetting composition.

Blending of the anti-blocking agent with the triggerable polymer in the binder composition may form a plurality of heterogeneous domains on the surface of a wet wipe upon drying. These heterogeneous binder domains refer to regions or areas of triggerable polymer on the surface of the wet wipe in which subregions or particles of the anti-blocking agent are present. Since the T_{g} or Tₘ of the anti-blocking agent may desirably be above that of the ambient temperature of the wet wipe during storage, the polymer molecules of the anti-blocking agent may desirably possess insufficient mobility to entangle, weld or interact with other polymer molecules of heterogeneous binder domains on adjacent wet wipe surfaces. Thus, contact between heterogeneous domains on adjacent wet wipe surfaces may result in decreased welding between these surfaces, since one anti-blocking agent sub-region will not substantially weld to another such region or to the triggerable polymer region of an opposing heterogeneous binder domain, due to the elevated T_{g} or Tₘ of the anti-blocking agent. Thus the anti-blocking agent may serve to interfere with the welding that is believed to occur when homogenous domains of triggerable polymer are present. Preferably, the amount of anti-blocking agent in the binder composition required to reduce the sheet-to-sheet adhesion is minimized, particularly in the case of high T_{g} or Tₘ materials, since they may contribute little to the in-use wet strength of the wet wipe in the binder composition. As such, inefficient anti-blocking agents which require high amounts of the anti-blocking agent in the binder composition may result in lower in-use wet strength of the binder composition, thereby requiring undesirably higher triggerable binder contents in the nonwoven web.

When an anti-blocking coating is selectively applied to the surface of the fibrous substrate, regions of anti-blocking coating are created. When the nonwoven material comprises the nonwoven web, the antiblocking coating may or may not be in contact with regions of triggerable polymer. These regions of anti-blocking coating may similarly interfere with the welding interaction of the triggerable polymer and may do so more effectively than the anti-blocking agent, as the anti-blocking coating may be located preferentially on the surface of the nonwoven web. In the case where both an anti-blocking agent and anti-blocking coating are employed, the regions of anti-blocking coating may not necessarily be coincident with the heterogeneous binder domains of the nonwoven web. Such an arrangement could result in increased interference with possible welding of the triggerable polymer, compared to the welding that may occur when the anti-blocking agent or anti-blocking coating is used alone.

The sheet-to-sheet adhesion experienced with dispersible wet wipes derived from nonwoven webs does not appear to become evident immediately or even soon after the wetting composition is applied to the nonwoven material. Sufficient time appears to be required for interactions between the triggerable polymer to occur and result in significant sheet-to-sheet adhesion so that it negatively impacts wet wipe dispensing. For example, immediately or soon after application of the wetting composition to the nonwoven material, placing at least two wet wipe surfaces in contact (e.g. such as by folding or stacking), and application of appropriate pressure, the sheet-to-sheet adhesion is not of sufficient magnitude to negatively impact dispensing. A timeframe comparable to about a day is required for the sheet-to-sheet adhesion to increase to a sufficient magnitude. Thus, test methods for determining the ability of the anti-blocking agent or the anti-blocking coating to mitigate the sheet-to-sheet adhesion that are performed 1) immediately or soon after application of the wetting composition to the nonwoven web or 2) immediately or soon after the wet wipe surfaces are placed in contact, would not be able to appropriately differentiate suitable anti-blocking agents or coatings or application methods for these materials. Aging of the wet wipe stack or roll is required to appropriately identify suitable anti-blocking agents and coatings.

The anti-blocking agent and anti-blocking coating may desirably provide sheet-to-sheet adhesion values between two adjacent wet wipe surfaces of less than about 7 g/in, more desirably of less than about 5 g/in, even more desirably of less than about 3 g/in. Sheet-to-sheet adhesion may be measured according to the methods described herein for packaged wet wipes.

As previously discussed, it is believed that the amount of sheet-to-sheet adhesion may depend on several factors, including: (1) the T_{g} of the triggerable polymer in the wet wipe, (2) the storage temperature, (3) the applied pressure; and/or (4) the length of storage before use. As the ambient temperature in typical households and retail displays is about 23 °C, the T_{g} or Tₘ of the anti-blocking agent and anti-blocking coating may be selected to be at least ambient temperature or higher.

According to the present invention the anti-blocking agent and the anti-blocking coating have a T_{g} of at least about 23°C. In some embodiments, the anti-blocking agent and the anti-blocking coating have a T_{g} of at least about 25°C. In some embodiments, the anti-blocking agent and the anti-blocking coating have a T_{g} of at least about 27°C. In some embodiments, the anti-blocking agent and the anti-blocking coating have a T_{g} of at least about 30°C. In some embodiments, the anti-blocking agent and the anti-blocking coating have a T_{g} of at least about 35°C. In some embodiments, the anti-blocking agent and the anti-blocking coating have a T_{g} of at least about 40°C. In some embodiments, the anti-blocking agent and the anti-blocking coating have a T_{g} of at least about 45°C. In some embodiments, the anti-blocking agent and the anti-blocking coating have a T_{g} of at least about 50°C. In some embodiments, the anti-blocking agent and the anti-blocking coating have a T_{g} of at least about 55°C. In some embodiments, the anti-blocking agent and the anti-blocking coating have a T_{g} of at least about 60°C. In some embodiments, the anti-blocking agent and the anti-blocking coating have a T_{g} of at least about 65°C. In some embodiments, the anti-blocking agent and the anti-blocking coating have a T_{g} of at least about 70°C. In some embodiments, the anti-blocking agent and the anti-blocking coating have a T_{g} of at least about 75°C. In some embodiments, the anti-blocking agent and the anti-blocking coating have a T_{g} of at least about 80°C. In some embodiments, the anti-blocking agent and the anti-blocking coating have a Tg of at least about 85°C. In some embodiments, the anti-blocking agent and the anti-blocking coating have a T_{g} of at least about 90°C. In some embodiments, the anti-blocking agent and the anti-blocking coating have a T_{g} of at least about 95°C. In some embodiments, the anti-blocking agent and the anti-blocking coating have a T_{g} of at least about 100°C. In some embodiments, the anti-blocking agent and the anti-blocking coating have a T_{g} of at least about 105°C. In some embodiments, the anti-blocking agent and the anti-blocking coating have a T_{g} of between about 23°C and about 105°C, including any integer value and fractional value there between.

According to the present invention the anti-blocking agent and the anti-blocking coating have a Tₘ of at least about 23°C. In some embodiments, the anti-blocking agent and the anti-blocking coating have a Tₘ of at least about 25°C. In some embodiments, the anti-blocking agent and the anti-blocking coating have a Tₘ of at least about 27°C. In some embodiments, the anti-blocking agent and the anti-blocking coating have a Tₘ of at least about 30°C. In some embodiments, the anti-blocking agent and the anti-blocking coating have a Tₘ of at least about 35°C. In some embodiments, the anti-blocking agent and the anti-blocking coating have a Tₘ of at least about 40°C. In some embodiments, the anti-blocking agent and the anti-blocking coating have a Tₘ of at least about 45°C. In some embodiments, the anti-blocking agent and the anti-blocking coating have a Tₘ of at least about 50°C. In some embodiments, the anti-blocking agent and the anti-blocking coating have a Tₘ of at least about 55°C. In some embodiments, the anti-blocking agent and the anti-blocking coating have a Tₘ of at least about 60°C. In some embodiments, the anti-blocking agent and the anti-blocking coating have a Tₘ of at least about 65°C. In some embodiments, the anti-blocking agent and the anti-blocking coating have a Tₘ of at least about 70°C. In some embodiments, the anti-blocking agent and the anti-blocking coating have a Tₘ of at least about 75°C. In some embodiments, the anti-blocking agent and the anti-blocking coating have a Tₘ of at least about 80°C. In some embodiments, the anti-blocking agent and the anti-blocking coating have a Tₘ of at least about 85°C. In some embodiments, the anti-blocking agent and the anti-blocking coating have a Tₘ of at least about 90°C. In some embodiments, the anti-blocking agent and the anti-blocking coating have a Tₘ of at least about 95°C. In some embodiments, the anti-blocking agent and the anti-blocking coating have a Tₘ of at least about 100°C. In some embodiments, the anti-blocking agent and the anti-blocking coating have a Tₘ of at least about 105°C. In some embodiments, the anti-blocking agent and the anti-blocking coating have a Tₘ of at least about 110°C. In some embodiments, the anti-blocking agent and the anti-blocking coating have a Tₘ of at least about 115°C. In some embodiments, the anti-blocking agent and the anti-blocking coating have a Tₘ of at least about 120°C. In some embodiments, the anti-blocking agent and the anti-blocking coating have a Tₘ of at least about 125°C. In some embodiments, the anti-blocking agent and the anti-blocking coating have a Tₘ of at least about 130°C. In some embodiments, the anti-blocking agent and the anti-blocking coating have a Tₘ of at least about 135°C. In some embodiments, the anti-blocking agent and the anti-blocking coating have a Tₘ of at least about 140°C. In some embodiments, the anti-blocking agent and the anti-blocking coating have a Tₘ of between about 23°C and about 140°C, including any integer value and fractional value there between.

The anti-blocking agent and the anti-blocking coating may be selected from a variety of polymers which have the desired T_{g} or Tₘ as discussed above. In a preferred embodiment, the anti-blocking agent and the anti-blocking coating may be polymer dispersions that exist in water as stabilized polymer particles.

Preferred polymeric materials suitable for use as either an anti-blocking agent or anti-blocking coating include RESYIN® 225-A (Vinamul Polymers of Bridgewater, N.J), DUR-O-SET® SBX (Vinamul Polymers of Bridgewater, NJ), RESYN® SB-321 (Vinamul Polymers of Bridgewater, NJ), DUR-O-SET® TX-800, RESYN® 1971 (Vinamul Polymers of Bridgewater, NJ). Particularly preferred polymeric materials suitable for use as either an anti-blocking agent or anti-blocking coating are RHOPLEX® B-88 and RHOPLEX® ECO-4015, both available from Rohm and Haas Company of Philadelphia, PA. Preferred polymeric materials suitable for use as an anti-blocking coating include VINAC® 21, VINAC® 911, VINAC® 9100, AIRFLEX® 4530, VINAC® XX210, all available from Air Products Polymers, L.P. of Allentown, PA. Additional preferred polymeric materials for use as the anti-blocking coating include RESYN® 1025, RESYN® 1072, RESYN® 1601, all available from Vinamul Polymers of Bridgewater, NJ. Another preferred polymeric material for use as the anti-blocking coating is LATRIX® 6330, which is a styrene-butadiene copolymer emulsion available from Nalco Chemical Co. of Naperville, IL. Particularly preferred polymeric materials suitable for use as an anti-blocking coating are the aqueous thermoplastic polymer dispersions disclosed in US Serial Number 10/925693.

### Wetting Composition

The wetting composition for use in combination with the nonwoven materials may desirably comprise an aqueous composition containing the insolubilizing agent that maintains the coherency of the binder composition and thus the in-use strength of the wet-wipe until the insolubilizing agent is diluted with tap water. Thus the wetting composition may contribute to the triggerable property of the triggerable polymer and concomitantly the binder composition.

The insolubilizing agent in the wetting composition can be a salt, such as those previously disclosed for use with the ion-sensitive polymer, a blend of salts having both monovalent and multivalent ions, or any other compound, which provides in-use and storage strength to the binder composition and may be diluted in water to permit dispersion of the wet wipe as the binder composition transitions to a weaker state. The wetting composition may desirably contain more than about 0.3 weight percent of an insolubilizing agent based on the total weight of the wetting composition. The wetting composition may desirably contain from about 0.3 weight percent to about 10 weight percent of an insolubilizing agent based on the total weight of the wetting composition. More desirably, the wetting composition may contain from about 0.5 weight percent to about 5 weight percent of an insolubilizing agent based on the total weight of the wetting composition. More desirably, the wetting composition may contain from about 1 weight percent to about 4 weight percent of an insolubilizing agent based on the total weight of the wetting composition. Even more desirably, the wetting composition may contain from about 1 weight percent to about 2 weight percent of an insolubilizing agent based on the total weight of the wetting composition.

The wetting composition may desirably be compatible with the triggerable polymer, the cobinder polymer, the anti-blocking agent and any other components of the binder composition. In addition, the wetting composition desirably contributes to the ability of the wet wipes to maintain coherency during use, storage and/or dispensing, while still providing dispersibility in tap water.

The wetting composition may include a variety of additives or components, including those disclosed in U.S. Patent Publication No. 2002/0155281. Possible additives may include, but are not limited to skin-care additives, odor control additives, wetting agents and/or cleaning agents; surfactants, pH control agents, preservatives and/or anti-microbial agents.

The wet wipes, as disclosed herein, do not require organic solvents to maintain in-use strength, and the wetting composition may be substantially free of organic solvents. Organic solvents may produce a greasy after-feel and cause irritation in higher amounts. However, small amount of organic solvents may be included in the wetting composition for different purposes other than maintaining in-use wet strength. In one embodiment, small amounts of organic solvents (less than about 1%) may be utilized as fragrance or preservative solubilizers to improve process and shelf stability of the wetting composition. The wetting composition may desirably contain less than about 5 weight percent of organic solvents, such as propylene glycol and other glycols, polyhydroxy alcohols, and the like, based on the total weight of the wetting composition. More desirably, the wetting composition may contain less than about 3 weight percent of organic solvents. Even more desirably, the wetting composition may contain less than about 1 weight percent of organic solvents.

Relative to the weight of the dry substrate, the wet wipe may desirably contain from about 10 percent to about 600 percent of the wetting composition by weight, more desirably from about 50 percent to about 500 percent of the wetting composition by weight, even more desirably from about 100 percent to about 400 percent of the wetting composition by weight, and especially more desirably from about 200 to 300 percent of the wetting composition.

### Method of Making Wet Wipes

The binder composition may be applied to the fibrous material by any known process. Suitable processes for applying the binder composition include, but are not limited to printing, spraying, electrostatic spraying, the use of metered press rolls or impregnating. The amount of binder composition may be metered and distributed uniformly onto the fibrous material or may be non-uniformly distributed onto the fibrous material.

For ease of application, the binder composition may be applied to the fibrous material in combination with a solvent, as a solution or mixture. A variety of solvents may be used, including, for example, water, methanol, ethanol, acetone, or the like, with water being the preferred solvent. The amount of binder composition in the solvent may vary; depending on a variety of factors, including the identity and physical characteristics of the triggerable polymer, the cobinder, and/or the anti-blocking agent that are being used, as well as the identity and physical characteristics of the fibrous material to which the binder composition is being applied. Desirably, the mixture or solution of the binder composition may contain up to about 50 percent by weight of binder composition solids. More desirably, the binder solution or mixture may contain from about 10 to 30 percent by weight of binder composition solids. Even more desirably, the binder solution or mixture may contain about 12 to 25 percent by weight binder composition solids.

Once the binder composition is applied to the fibrous material, drying, if necessary, may be achieved by any conventional means. Once dry, the nonwoven material may exhibit improved tensile strength when compared to the tensile strength of the untreated wet-laid or dry-laid fibrous material, and yet should have the ability to rapidly "fall apart" or disintegrate when placed in tap water.

A number of techniques may be employed to manufacture the wet wipes. In one embodiment, these techniques may include the following steps:
1. Providing the fibrous material (e.g., an unbonded airlaid, a tissue web, a carded web, fluff pulp, etc.).
2. Applying the binder composition to the fibrous material, typically in the form of a liquid, suspension, or foam to provide the nonwoven web
3. The nonwoven web may be dried.
4. The nonwoven web may be coated with a antiblocking coating composition in the form of a liquid, suspension, or foam.
5. Applying a wetting composition to the nonwoven web to generate the wet wipe.
6. Placing the wet wipe in roll form or in a stack and packaging the product.

In one embodiment, Step 2 as discussed above, may be carried out such that the triggerable polymer and the anti-blocking agent of the binder composition are applied as a mixture to the fibrous material, referred to as mixture application.

In another embodiment, Step 2 as discussed above, the application of the binder composition may be achieved by applying the triggerable polymer and the anti-blocking agent via different spray booms that are arranged sequentially, such that the triggerable polymer is applied first and the anti-blocking agent is applied second. This application technique may be referred to as a tandem or sequential application. That is, the fibrous material may travel past a plurality of spray booms, wherein a first set of spray booms applies the triggerable polymer and the second set of spray booms applies the anti-blocking agent, or vice-versa. This application technique may produce a layering effect of the triggerable polymer and the anti-blocking agent, preferably concentrating the anti-blocking agent on the surface of the nonwoven web.

In one embodiment, the binder composition as applied in step 2 may comprise the triggerable polymer. In another embodiment, the binder composition as applied in step 2 may comprise the triggerable polymer and the anti-blocking agent. In a further embodiment, the binder composition as applied in step 2 may comprise the triggerable polymer and the cobinder. Following step 3, the anti-blocking coating may be applied. Application of the anti-blocking coating may be achieved using a variety of techniques, including gravure printing, flexographic printing, inkjet printing, spray application and foam application, for example.

Wipes may also be prepared by applying the binder composition to the fibrous material, followed by drying, application of the anti-blocking coating (if desired) and winding of the resulting nonwoven web into a roll. In this embodiment, the wetting composition may be added some time later. For example, large rolls of the dry nonwoven web may be prepared as an intermediate material. This procedure may be advantageous as part of the manufacturing process. It may be desirable that blocking of the dry rolls or stacks of nonwoven web does not occur during storage, as such an occurrence would negatively impact unwinding of the rolls and subsequent converting of the dry basesheet into a wet wipe. Dry blocking can occur when the T_{g} of the binder composition in a nonwoven material is below or close to the storage temperature of the dry rolls of nonwoven materials. In addition to improving wet wipe blocking and dispensing, the anti-blocking agents and anti-blocking coatings described herein reduce dry roll sheet-to-sheet adhesion (blocking) as well.

The finished wet wipes may be individually packaged, desirably in a folded condition, in a moisture proof envelope or packaged in containers holding any desired number of sheets in a water-tight package with a wetting composition applied to the wipe. Some example processes which can be used to manufacture folded wet wipes are described in U.S. Patent Nos. 5,540,332 and 6,905,748.
The finished wipes may also be packaged as a roll of separable sheets in a moisture-proof container holding any desired number of sheets on the roll with a wetting composition applied to the wipes. The roll can be coreless and either hollow or solid. Coreless rolls, including rolls with a hollow center or without a solid center, can be produced with known coreless roll winders, including those of SRP Industry, Inc. (San Jose, CA); Shimizu Manufacturing (Japan), and the devices disclosed in U.S. Pat. No. 4,667,890 . The U.S. Pat. No. 6,651,924 also provides examples of a process for producing coreless rolls of wet wipes.

### Wet Wipe Properties

The wet wipes , as disclosed herein, desirably may be made to have sufficient in-use wet tensile strength, wet thickness, opacity, and dispersibility. They may also be made to be usable without breaking or tearing, to be consumer acceptable, and provide problem-free disposal once disposed in a household sanitation system.

The wet wipe as disclosed herein desirably may have an in-use wet strength ranging from at least about 100 g/in to about 1000 g/in. More desirably, the wet wipe may have an in-use wet strength ranging from at least about 200 g/in to about 800 g/in. Even more desirably, the wet wipe may have an in-use wet strength ranging from at least about 300 g/in to about 600 g/in. Most desirably, the wet wipe may have an in-use wet strength ranging from at least about 350 g/in to about 550 g/in.

The wet wipe may be configured to provide all desired physical properties by use of a single or multi-ply wet wipe product, in which two or more plies of nonwoven material are joined together by methods known in the art to form a multi-ply wipe.

The total basis weight of the nonwoven material, consisting of a single or multiple layers of nonwoven material in the final wet wipe product, may be in the range of at least about 25 gsm to about 120 gsm. More desirably, the basis weight of the nonwoven material may be between about 40 gsm and 90 gsm. Even more desirably, the basis weight of the nonwoven material may be between about 60 gsm and 80 gsm. Especially more desirably, the basis weight of the nonwoven material may be between about 70 and 75 gsm.

The wet opacity of the wet wipe, or the tendency of the wet wipe to prevent the transmission of light, may desirably be higher (i.e. less transmitted light) as it provides an indication that the wet wipe will be able to perform its desired function without breaking or tearing. Desirably, the wet wipe, as disclosed herein, may have a wet opacity greater than about 20%. More desirably, the wet wipe may have a wet opacity greater than about 35%. Even more desirably, the wet wipe may have a wet opacity greater than about 45%.

Preferably, as described earlier, the sheet-to-sheet adhesion of the wet wipe in the final packaged product may be lower, in able to provide easier dispensing of the wet wipe. Accordingly, the wet wipes, as disclosed herein, may desirably have a sheet-to-sheet adhesion less than about 7 g/in. More desirably, the wet wipes may have a sheet-to-sheet adhesion less than about 5 g/in. Even more desirably, the wet wipes may have a sheet-to-sheet adhesion less than about 3 g/in.

The average thickness of the wet wipe may be in the range of at least about 0.25 mm to about 1.5 mm. More desirably, the average thickness of the wet wipe may be between 0.3 mm and 1.0 mm. Even more desirably, the average thickness of the wet wipe may be between 0.5 mm and 1.0 mm.

As mentioned previously, the wet wipes, as disclosed herein, may be sufficiently dispersible so that they lose enough strength to break apart in tap water under conditions typically experienced in household or municipal sanitation systems. Also mentioned previously, the tap water used for measuring dispersibility should encompass the concentration range of the majority of the components typically found in the tap water compositions that the wet wipe would see upon disposal. Previous methods for measuring dispersibility of the nonwoven materials whether dry or pre-moistened, have commonly relied on systems in which the material was exposed to shear while in water, such as measuring the time for a material to break up while being agitated by a mechanical mixer. Constant exposure to such relatively high, uncontrolled shear gradients offers an unrealistic and overly optimistic test for products designed to be flushed in a toilet, where the level of shear is extremely weak or brief. Shear rates may be negligible, for example once the material enters a septic tank. Thus, for a realistic appraisal of wet wipe dispersibility, the test methods should simulate the relatively low shear rates the products will experience once they have been flushed in the toilet.

A static soak test, for example, should illustrate the dispersibility of the wet wipe after it is fully wetted with water from the toilet and where it experiences negligible shear, such as in a septic tank. Desirably, the wet wipe may have less than about 100 g/in of tensile strength after 5 h when soaked in water with a total dissolved solids up to 500 ppm and a CaCO₃ equivalent hardness up to about 250 ppm. More desirably, the wet wipe may have less than about 100 g/in of tensile strength after 3 h when soaked in water with a total dissolved solids up to 500 ppm and a CaCO₃ equivalent hardness up to about 250 ppm. Even more desirably, the wet wipe may have less than about 100 g/in of tensile strength after 1 h when soaked in water with a total dissolved solids up to 500 ppm and a CaCO₃ equivalent hardness up to about 250 ppm.

After flushing in the toilet in a household or building, the wet wipe may enter into the sanitary sewer system through pipes referred to as sewer laterals. In sewer laterals, the motion of the water typifies a "gentle sloshing" or wave-like motion. A "slosh box" is a box or a container that rocks back and forth with water inside, thereby creating a wave front and subjecting the wet wipe to intermittent motion that is capable of mimicking the "gentle sloshing" motion that the wet wipe would experience in sewer laterals. While the slosh box may be more vigorous than the actual action in a sewer lateral, the method is more representative of the lateral movement the wet wipe would experience than the higher shear methods described above. Desirably, the wet wipe will break-up in the slosh box to pieces of size less than about 1 inch square in area. Dispersion of the wet wipe to pieces of about this size or smaller may be sufficient to allow the pieces to pass through the bar screens typically found in municipal sanitary sewer treatment facilities and not cause problems or blockages in households.

In one embodiment, the wet wipe may break up into pieces of less than about 1 inch square in a slosh box in less than about 500 minutes in water with a total dissolved solids up to 500 ppm and a CaCO₃ equivalent hardness up to about 250 ppm. In another embodiment, the wet wipe may desirably break up into pieces of less than about 1 inch square in area in a slosh box in less than about 300 minutes in water with a total dissolved solids up to 500 ppm and a CaCO₃ equivalent hardness up to about 250 ppm. In a further embodiment, the wet wipe may more desirably break up into pieces of less than about 1 inch square in area in a slosh box in less than about 100 minutes in water with a total dissolved solids up to 500 ppm and a CaCO₃ equivalent hardness up to about 250 ppm. In another embodiment, the wet wipe may even more desirably break up into pieces of less than about 1 inch square in area in a slosh box in less than about 60 minutes in water with a total dissolved solids up to 500 ppm and a CaCO₃ equivalent hardness up to about 250 ppm.

Desirably, the wet wipes, as disclosed herein, may possess an in-use wet tensile strength of at least about 150 g/in when wetted with 10% to 400% of the wetting composition by weight relative to the weight of the nonwoven material, and a tensile strength of less than about 100 g/in when soaked in water with a total dissolved solids up to 500 ppm and a CaCO₃ equivalent hardness up to about 250 ppm after about 24 hours or less, desirably after about one hour.

Most desirably, the wet wipes, as disclosed herein, may possess an in-use wet tensile strength greater than about 300 g/in when wetted with 10% to 400% of the wetting composition by weight relative to the nonwoven material, and a tensile strength of less than about 100 g/in when soaked in water with a total dissolved solids up to 500 ppm and a CaCO₃ equivalent hardness up to about 250 ppm after about 24 hours or less, desirably after about one hour.

In a further embodiment, the wet wipes, as disclosed herein, may possess an in-use wet tensile strength greater than about 300 g/in when wetted with 10% to 400% of the wetting composition by weight relative to the weight of the nonwoven material, and a slosh box break-up time of less than about 300 minutes in water with a total dissolved solids up to 500 ppm and a CaCO₃ equivalent hardness up to about 250 ppm.

The wet wipe preferably maintains its desired characteristics over the time periods involved in warehousing, transportation, retail display and storage by the consumer. In one embodiment, shelf life may range from two months to two years.

The wet wipes, as disclosed herein, are illustrated by the following examples, which are not to be construed in any way as imposing limitations upon the scope thereof.

### General Procedures

### Wet Opacity Methods

The opacity of the premoistened wet wipes was measured with a BYK-Gardner Color-Guide Sphere Spin Spectrophotometer. The instrument uses a d/8° and 45/0 geometry (diffuse illumination at 8° and 45° angles). The opacity of the wet wipes are measured "as-is". The wet wipes are tested individually and they are first measured against a 100 mm × 100mm black standard and then against a 90 mm × 90 mm white standard. A Black Deldrin holder is used for total hemispherical reflectance of 380-760 nm at 10 nm intervals. The opacity is calculated and recorded from the digital readout on the spectrophotometer. Five wipes were tested individually in an identical manner and the results averaged.

### Lab Preparation of Airlaid Nonwoven Material Basesheets

A weak, 50 gsm, 1.0 mm thick thermally-bonded airlaid (TBAL) fibrous material was cut into 254 mm × 330 mm (10"x13") handsheets. The thermally-bonded airlaid material was prepared as described in U.S. Patent Application Publication No. 2004/0063888 in its entirety. Using various binder compositions at 15% total solids, the TBAL handsheets were treated with the binder composition using a pressurized spray unit to achieve a final 24% total content of binder composition in the handsheets.

The resulting lab-prepared airlaid nonwoven material basesheet was manually removed and dried in a Werner Mathis, Model LTV Through-Air Dryer (TAD) at 180°C for 23 seconds at 100% fan speed.

### Pilot-Scale Preparation Of Airlaid Nonwoven Web Basesheets

Basesheets of the nonwoven web, as used in some of the following tables, were formed continuously on a pilot-scale airlaid machine having a width of 610 mm (24 inches). A DanWeb airlaid former with two forming heads was utilized to produce the airlaid fibrous materials, from which basesheets of the nonwoven web were formed. Weyerhauser CF405 bleached softwood kraft fiber, in pulp sheet form, was fiberized in a hammermill and deposited onto a moving wire at 61-91.4 m/min (200-300 fpm).The fibrous material was densified to the desired level by heated compaction rolls and transferred to an oven wire, where it was sprayed on the top side with the desired binder composition, applying approximately half of the binder composition onto the dry fibrous material to provide a wet partially formed nonwoven web.

A series of Quick Veejet® nozzles, Nozzle type 800050, manufactured by Spraying Systems Co., Wheaton, III., operating at approximately 689.5 kPa (100 psi) were employed to spray the binder composition onto the fibrous material. A spray boom over the fibrous material utilized 5 such nozzles on 140 mm (5.5 inch) centers with a tip-to-wire distance of 203 mm (8 inches). This arrangement yielded 100% overlap of the spray cones of the binder composition. Each of the binder compositions as shown in the following tables was sprayed at approximately 15% binder solids with water as the carrier. The wet partially formed nonwoven web was carried through an oven section of approximately 9.14 m (30 feet) in length, operating at 202 °C (395 °F) to provide a dry partially formed nonwoven web. The dry partially formed nonwoven web was then turned over, transferred onto another wire and passed under a second spray boom to add the other half of the desired binder composition, for a total weight percent of 20-24% binder solids relative to the dry mass of the nonwoven web to provide a wet nonwoven web. The wet nonwoven web was then passed through a second oven section as described above, to complete the drying of the nonwoven web.

### Large-scale Preparation of Airlaid Nonwoven Web Basesheets

In this large scale process, a basesheet of airlaid nonwoven web was formed continuously on a commercial scale airlaid machine similar to the pilot-scale machine. Weyerhauser CF405 bleached softwood kraft fiber in pulp sheet form was used as the fibrous material. This airlaid fibrous material was densified to the desired level by heated compaction rolls and transferred to an oven wire, where it was sprayed on the top side with the desired binder composition, applying approximately half of the desired binder solids onto the dry fibrous material.

A series of Unijet® nozzles, Nozzle type 730077, manufactured by Spraying Systems Co., Wheaton, III., operating at approximately 482.6 - 827.4 kPa (70-120 psi) were used to spray the binder composition onto the fibrous material. Each binder composition was sprayed at approximately 15% binder solids with water as the carrier. The wet partially formed nonwoven web was carried through an oven operating at 177-204 °C (350-400 F) to provide the dry partially formed nonwoven web. The dry partially formed nonwoven web was then turned over, transferred onto another wire and passed under another three spray booms to add the other half of the desired binder composition, for a total weight percent of 20-24% binder solids relative to the dry mass of the nonwoven web. The nonwoven web was then passed through a second oven section as described above, to complete the drying of the nonwoven web.

### Lab-scale Offset Gravure Printing

An airlaid nonwoven web prepared on either the pilot-scale or large-scale airlaid machines was fed into a rubber-rubber nip of a rotogravure laboratory printer (available from RETROFLEX, INC, De Pere, WI) to apply the printing compositions to each side of the sample simultaneously. The gravure rolls were electronically engraved and had a volume of 12.4 million cubic microns (MCM) per mm² (8.0 billion cubic microns (BCM) per square inch) of roll surface. The rubber rolls had a 152 mm (6-inch) diameter with 9.5 mm (3/8 inch) thickness covered with a 75 Shore A durometer cast polyurethane supplied by American Roller Company. The gravure printer was run at a speed of 30.5 m/min (100 feet per minute). Additional details of the printing process are given in the example tables.

### Pilot-scale Gravure Printing

An airlaid nonwoven web, prepared on either the pilot-scale or large-scale airlaid machines, was fed into a rubber-rubber nip of a pilot-scale rotogravure printer to apply the printing compositions to each side of the sample simultaneously. Two different sets of electronically engraved gravure rolls were used in either a direct or offset configuration. The first set had one gravure roll with a volume of 12.4 MCM per mm² (8.0 BCM per square inch) of roll surface and the other having a volume of 10.9 MCM per mm² (7.0 BCM per square inch) of roll surface. The second set had one gravure roll with a volume of 6.2 MCM per mm² (4.0 BCM per square inch) of roll surface and the other having a volume of 7.8 MCM per mm² (5.0 BCM per square inch) of roll surface. The rubber rolls were a 75 Shore A durometer cast polyurethane supplied by American Roller Company. The gravure printer was run at a speed of 243.8 m/min (800 feet per minute). Additional details of the coating process are given in the example tables with regards to the particular gravure rolls utilized, the printing application solids, and the printer configuration.

### Large-scale Flexographic Printing

The nonwoven webs prepared on the large-scale airlaid machine were printed on both sides using two flexographic printing presses in consecutive applications. The nonwoven web was fed into a rubber-steel nip of the first press on the large-scale flexographic printer to apply the printing composition to side 1 of the nonwoven web. The nonwoven web continued through the printing process to a second rubber-steel nip of the second flexographic printing press where the printing composition was applied to side 2 of the nonwoven web. Both flexographic printing presses used laser engraved ceramic anilox rolls having a volume of 13.5 MCM per mm² (8.7 BCM per square inch) of roll surface. The anilox rolls were supplied by Harper Corporation of America. The rubber rolls were 55 Shore A durometer EPDM supplied by Rol-Tec, Green Bay, WI. The flexographic printer was run at a speed of 243.8 m/min (800 feet per minute). Additional information regarding the printing process is included with the example descriptions.

### Lab-Prepared Wet Wipe Preparation and Aging Protocol

Each 254 mm × 330 mm² (10"x13") lab-prepared airlaid nonwoven material was die cut into two 191 mm x 140 mm (7.5"×5.5") dry wipes, with the shorter direction being the machine-direction (MD) direction. Each dry wipe was then sprayed with a 250% add-on of a wetting composition that is used on commercially available wet wipes under the trade designation KLEENEX® COTTONELLE FRESH® Folded Wipes (Kimberly-Clark Corporation of Neenah, WI) but containing 2 wt% sodium chloride (insolubilizing agent) to yield lab-prepared wet wipes. A stack of 10 lab-prepared wet wipes was formed and placed inside a re-sealable plastic bag. The stack of 10 lab-prepared wet wipes in the re-sealable plastic bag was compressed using an Atlas laboratory wringer (Atlas Electric Devices Co. of Chicago, IL) with no additional load added. The compression of the stack of lab-prepared wet wipes by the Atlas laboratory wringer as utilized in this method was not sufficient to mimic the packaged wet wipe product sheet-to-sheet adhesion but rather generated sheet-to-sheet adhesion values that trended lower than that of improved methods described in the section *Wet Wipe Prototype Preparation and Aging Protocol.* However, the method used with the Atlas laboratory wringer does allow for relative differentiation of materials as being suitable as anti-blocking agents or anti-blocking coatings. The compressed stack of TBAL wet wipes was then aged under 1000 g of weight for 72 h. The un-weighted stack was then transferred to a 46 °C (115 F) oven for an additional 24 h before testing.

### Wet Wipe Prototype Preparation and Aging Protocol

A section of airlaid basesheet produced on either the pilot-scale or large-scale airlaid machine was randomly cut into 191 mm × 140 mm (7.5"×5.5") dry wipes (i.e., nonwoven material to which wetting composition has not been added), with the shorter direction being the machine direction of the basesheet. Ten of the dry wipes were wetted with 250% add-on of a wetting composition that is used on commercially available wet wipes under the trade designation KLEENEX® COTTONELLE FRESH® Folded Wipes (Kimberly-Clark Corporation of Neenah, WI) but containing 2 wt% sodium chloride. The 10 wet wipes were stacked, placed inside a re-sealable plastic bag and compressed by use of a 9.98 kg (22 lb) metal roller, and rolled four times in both the MD and CD directions. Compression of the wet-wipe prototypes by this method more effectively mimics the packaged wet wipe sheet-to-sheet adhesion than that of the method used for the lab-prepared wet wipes. The compressed stack of wet wipes was then aged under 1000 g of weight for 72 h. After removal of the weight, the stack was then transferred to a 46 °C oven for an additional 24 h before testing.

### Dry Basesheet Aging Method

Basesheet samples were cut into 76 mm (3") wide dry wipe samples in the MD direction and 152 mm (6") in length. Ten basesheet samples were paired into five stacked pairs and sandwiched between two Plexiglas plates and weighted with a 11.79 kg (26 pound) weight. The weighted samples were aged in a 60 °C oven for 1 hour. The weighted samples were removed from the oven and aged for an additional 24 h in TAPPI conditions before 180° t-peel measurements.

### Wet- Wipe Tensile Measurements

A SinTech 1/D tensile tester with Testworks 4.08 version software, and a 100 Newton load cell with pneumatic grips was utilized for all sample testing. In the case of lab-prepared wet wipes and wet wipe prototypes, the wet wipes were removed from the oven, allowed to cool, and after removal from the plastic bag the wet wipes were cut in the center in the MD or cross-deckle (CD) direction to yield 3 stacks of 25.4 mm - 140 mm (1"x5.5") wet wipe strips (25.4 mm - 191 mm (1"x7.5")), 10 layers thick.

In the case of packaged wet wipe product testing, the wet wipes were removed from the package, with 25.4 mm (1") wide strips cut from the center of the wipes in the specified MD or CD direction. Tensile strips were cut from at least 12 randomly selected wipes from the wet wipe packages.

### 180°T-Peel Measurements (Sheet-to-Sheet Adhesion)

A 180° t-peel measurement was used to determine the sheet-to-sheet adhesion between adjacent wet wipe surfaces and adjacent dry nonwoven material surfaces. The method for the 180° t-peel measurement is based upon ASTM D1876-01 *Standard Test Method for Peel Resistance of Adhesives (T-Peel Test)* with the following modifications. A crosshead speed of 508 mm/min (20 inches/minute) with a gauge length of 38 mm (1.5 inches) was used for all measurements. Measurements were recorded between 12.7 mm and 152 mm (0.5 inches and 6.0 inches) with the end test point at 165 mm (6.5 inches). Lab-prepared wet wipes were aged prior to measurement according to the "Lab-prepared Wet Wipe Preparation and Aging Protocol". Wet Wipe Prototypes were aged prior to measurement according to the "Wet Wipe Prototype Preparation and Aging Protocol". Packaged wet wipes were used as received. Packaged wet wipes as disclosed herein were aged at ambient temperature for at least 30 days before testing. Commercially obtained wet wipes were used "as received." In the case of wet wipes, the aged wipes were cut into samples 25.4 mm (1" (in.)) width and a depth of at least two layers thick, with a sample size of ten used for measurement. In the case of dry basesheet, the aged basesheet measured 76 mm (3") width, with a sample size of six used for measurement.

### Wet-Wipe In-Use Tensile Strength Measurements

In-use wet tensile and residual soak tensile measurements were determined using a pneumatic grip gauge separation of 76 mm (3") and a crosshead speed of 254 mm/min (10"/min). The peak load values g/mm (g/in.)) of at least 10 sample replicates were recorded and averaged and reported as machine-direction wet tensile strength (MDWT) or cross-deckle wet tensile strength (CDWT), depending on how the test samples were prepared.

### Wet-Wipe Dispersibility - Soak Measurements

Dispersibility of the wet wipes was gauged by soaking the wet wipe strips in a defined volume of an aqueous solution. The volume (mL) of the aqueous solution was adjusted to equal 410 mL per wet wipe strips. For example, 10 wet wipe strips require 4100 mL of aqueous test solution. The wet wipe strips were soaked in the aqueous test solution for set periods of time, typically 1, 3, or 5 h before residual soak strength measurements were recorded using the tensile method described for the *Wet-Wipe In-Use Tensile Strength Measurements.* Three aqueous test solutions were utilized: 1) deionized water; 2) a tap water solution containing about 112 ppm HCO₃⁻, 66 ppm Ca²⁺, 20 ppm Mg²⁺, 65 ppm Na⁺, 137 ppm Cl⁻, 100 ppm SO₄²⁻ with a total dissolved solids of 500 ppm and a calculated water hardness of about 248 ppm equivalents CaCO₃; and 3) a "soft water" solution containing about 6.7 ppm Ca²⁺, 3.3 ppm Mg²⁺, and 21.5 ppm Cl⁻ with a total dissolved solids of 31.5 ppm and a calculated water hardness of about 30 ppm equivalents CaCO₃. The "lab-prepared wet wipes" and "wet wipe prototypes" were evaluated in the static soak tests with deionized water. The "packaged wet wipes" were evaluated with the "soft water" and "tap water" solutions.

### Packaged Wet Wipe Dispersibility - Slosh Box Measurements

The slosh box used for the dynamic break-up of the wet wipes consists of a 356 mm W × 457 mm D × 305 mm H (14"W×18"D×12"H) plastic box constructed from 12.7 mm (0.5") thick Plexiglas with a tightly fitting lid. The box rests on a platform, with one end attached to a hinge and the other end attached to a reciprocating cam. The amplitude of the rocking motion of the slosh box is ± 50.8 mm (101.6 mm range) (±2" (4" range)). The speed of the sloshing action is variable but was set to a constant speed of 20 revolutions per minute of the cam, or 40 sloshes per minute. A volume of 2000 mL of either the "tap water" or "soft water" soak solution was added to the slosh box before testing. A packaged wet wipe was randomly selected from the stack or roll and unfolded. The slosh box was started and timing was started once the wet wipe was added to the soak solution. The break-up of the wet wipe in the slosh box was visually observed and the time required for break-up into pieces less than about 1" square in area was recorded. At least three replicates of the samples were recorded and averaged to achieve the recorded values. Samples which did not break-up into pieces less than about 25.4 mm (1" ) square in area within 24 h in a particular soak solution were considered non-dispersible in that soak solution by this test method.

### EXAMPLES

### Experimental Data

Table 1 provides comparative data including sheet-to-sheet adhesion values, in-use strengths and soak strengths for lab-prepared wet wipes generated from TBAL handsheets with binder compositions comprising combinations of a cationic ion-sensitive polyacrylate (the triggerable polymer) and a polymer additive, all of which are polymer emulsion materials. The cationic ion-sensitive polyacrylate described in the following examples is a copolymer of methyl acrylate (96 mol%) and [(2-acryloyloxy)ethyl] trimethyl ammonium chloride (4 mol%) with a weight average molecular weight between 140,000 to 200,000 g/mol as determined by gel permeation chromatography in a dimethylformamide/LiCl mobile phase.

Entry A in Table 1 illustrates a wet wipe containing only the cationic ion-sensitive polyacrylate in the binder composition with no added antiblocking agent or cobinder. The wet wipe possesses high strength with the applied wetting composition and demonstrates continued strength loss after soaking in deionized (DI) water from 1 to 3 hours. The wet wipe of entry A has comparatively high sheet-to-sheet adhesion. Entries 1-3 demonstrate results where the binder composition of the lab-prepared wet wipes comprises between 65-85% cationic ion-sensitive polyacrylate with between 15-35% of RHOPLEX® B-88 (Rohm and Haas, Inc. of Philadelphia, PA), which is a nonionic surfactant stabilized acrylic emulsion with a T_{g} of +91 °C. RHOPLEX® B-88 is an effective anti-blocking agent. When 15% of the antiblocking agent was used in the binder composition, a drop in sheet-to-sheet adhesion to 57% of entry A was observed, with a further drop in sheet-to-sheet adhesion of 29% of entry A being observed with 35% of the Rhoplex® B-88 used in the binder composition.

Entries 4 and 5 illustrates results of lab-prepared wet wipes where the binder composition comprises between 65-75% cationic ion-sensitive polyacrylate with either 25% (entry 4) or 35% (entry 5) of RESYN® 225-A (Vinamul Polymers of Bridgewater, NJ), which is a cationic poly(vinyl acetate) emulsion with a T_{g} of +30 °C. Similar to the RHOPLEX® B-88, the RESYN® 225-A, with a T_{g} slightly higher than ambient temperature, demonstrates a significant drop of the sheet-to-sheet adhesion values at 25 to 35% in the binder composition. Some loss of in-use wet strength and dispersibility (higher residual soak strengths) is also observed at the higher levels of this anti-blocking agent.

Entry B illustrates an example of lab-prepared wet wipes with a binder composition containing 65% cationic ion-sensitive polyacrylate and 35% of AIRFLEX® 110 (Air Products Polymers, L.P. of Allentown, PA), which is a nonionic, vinyl acetate-ethylene copolymer with a T_{g} of +4 °C. A small decrease in sheet-to-sheet adhesion of only 71 % compared to entry A is observed with the AIRFLEX® 110 due to its low T_{g} which is also very inefficient as a relatively high content of 35% of the additive is required to achieve this value.

Entries C, D, and E illustrate examples of lab-prepared wet wipes with 35% of either PRINTRITE® 591 (T_{g} -10 °C), PRINTRITE® 595 (T_{g} -20 °C), and HYCAR® 9323N, (T_{g} -20 °C), respectively, in the binder composition, all of which are nonionic acrylic emulsions available from Noveon, Inc. of Cleveland Ohio. These three acrylic emulsion additives demonstrate sheet-to-sheet adhesion values close to or greater than the wet wipe of entry A due to the significantly low T_{g}s of these materials. A significant loss of in-use strength is also observed with these materials. These entries demonstrate that significantly low T_{g} amorphous polymeric materials are not effective anti-blocking agents. **Table 1.**

| Entry | Polymer Additive | Polymer Additive T_{g} (°C) | Polymer Additive Content in Binder Composition (%) | Sheet-to-sheet Adhesion g/mm (g/in) | Machine Direction Wet Tensile g/mm (g/in) | | |
|---|---|---|---|---|---|---|---|
| | | | | | In-Use MDWT | Residual MD Soak Strength in DI water, 1 h | Residual MD Soak Strength in DI water, 3 h |
| A | none | N/A | 0 | 0.28 (7) | 17.4 | 4.1 (103) | 2.8 (71) |
| | | | | (100%)^{c} | (441) | (23%)^{d} | (16%)^{d} |
| 1 | Rhoplex® B-88 | | 15 | 0.16 (4) | 14.1 | 2.6 (65) | 1.2 (31) |
| | | | | (57%)^{c} | (359) | (18%)^{d} | (9%)^{d} |
| 2 | | +91 | 25 | 0.12 (3) | 17.9 | 3.7 (95) | 1.4 (35) |
| | | | | (43%)^{c} | (454) | (21%)^{d} | (8%)^{d} |
| 3 | | | 35 | 0.08 (2) | 14.6 | 4.7 (119) | 3.2 (81) |
| | | | | (29%)^{c} | (372) | (32%)^{d} | (22%)^{d} |
| 4 | Resyn® 225-A | | 25 | 0.16 (4) | 14.1 | 5.0 (126) | 3.9 (98) |
| | | | | (57%)^{c} | (359) | (35%)^{d} | (27%)^{d} |
| 5 | | +30 | 35 | 0.08 (2) | 13.1 | 5.8 (148) | 4.4 (112) |
| | | | | (29%)^{c} | (334) | (44%)^{d} | (33%)^{d} |
| B | Airflex® | +4 | 35 | 0.20 (5) | 17.5 | 5.2 (133) | 3.2 (82) |
| | 110 | | | (71%)^{c} | (445) | (30%)^{d} | (18%)^{d} |
| C | PrintRite® | -10 | 35 | 0.35 (9) | 9.5 | 4.0 (102) | 2.7 (68) |
| | 591 | | | (129%)^{c} | (241) | (42%)^{d} | (28%)^{d} |
| D | PrintRite® | -20 | 35 | 0.24(6) | 9.6 | 3.5 (89) | 2.7 (69) |
| | 595 | | | (86%)^{c} | (243) | (37%)^{d} | (28%)^{d} |
| E | Hycar® T- | -20 | 35 | 0.28 (7) | 10.6 | 4.3 (110) | 3.5 (90) |
| | 9323N | | | (100%)^{c} | (269) | (41%)^{d} | (33%)^{d} |

| | | | | | | | |
|---|---|---|---|---|---|---|---|
| ^{a}The numbered entries refer to the TBAL wet wipes employing anti-blocking agent; ^{b}The entries with letter designators refer to comparative examples; ^{c}Percentage ratio of example sheet-to-sheet adhesion to that of Entry A; ^{d}Percentage ratio of residual soak strength at either 1 or 3 h to that of the in-use strength. | | | | | | | |

Table 2 provides sheet-to-sheet adhesion values, in-use strengths and soak strengths for wet-wipe prototypes prepared from pilot-scale airlaid nonwoven web basesheets produced using either the cationic ion-sensitive polyacrylate alone or in combination with the Rhoplex® B-88 anti-blocking agent in the binder composition. In Entry F, a binder composition consisting of only the cationic ion-sensitive polyacrylate was sprayed onto the airlaid fibrous material. In both entries 6 and 7, the binder composition consists of 80 wt% cationic ion-sensitive polyacrylate and 20 wt% anti-blocking agent applied onto the basesheet at 15% spray solids. In Entry 7, the cationic ion-sensitive polyacrylate and the anti-blocking agent were applied as a mixture to the basesheet. In Entry 6 (tandem application), the cationic ion-sensitive polyacrylate and the anti-blocking agent were applied using the same spraying system described above in the section entitled *Pilot-Scale Preparation of Airlaid Nonwoven Web Basesheets.* However, in Entry 6 the cationic ion-sensitive polyacrylate and the anti-blocking agent were applied using different spray booms. In fact, the cationic ion-sensitive polyacrylate was applied using a first set of spray booms and the anti-blocking agent was applied using a second set of spray booms. The cationic ion-sensitive polyacrylate and anti-blocking agent were pumped at flow rates to maintain the same binder composition of Entry 7. The tandem addition method of the antiblocking agent demonstrates a more effective sheet-to-sheet adhesion reduction than the mixture addition method as demonstrated by a decrease of sheet-to-sheet adhesion of 56% (entry 6) versus 67% relative to the control, entry F, where no anti-blocking agent was used. Dispersibility is maintained, as illustrated by the low residual soak strength of 20-30% of the initial in-use wet-tensiles strength.

**Table 2**

| Entry ^{a.b} | Basis Weight (gsm) | Dry Caliper (mm) | Anti-blocking Agent | Binder Composition Content in Nonwoven Web (%) | Anti-Blocking Agent Addition Method | Anti-blocking Agent Content in Binder Composition (%) | Sheet-to-sheet Adhesion g/mm (g/in) | Machine Direction Wet Tensile g/mm (g/in) | |
|---|---|---|---|---|---|---|---|---|---|
| | | | | | | | | In-Use MDWT | Residual MD Soak Strength in DI water, I h |
| F | 60 | 1.22 | none | 20 | N/A | 0 | 0.35 (9) | 14.5 | 5.2 (133) |
| | | | | | | | (100%)^{c} | (368) | (36%)^{d} |
| 6 | 62 | 1.06 | Rhoplex® B-88 | 24 | Tandem | 20 | 0.20 (5) | 13.5 | 3.9 (99) |
| | | | | | | | (56%)^{c} | (344) | (29%)^{d} |
| 7 | 60 | 1.06 | Rhoplex® B-88 | 24 | Mixture | 20 | 0.24 (6) | 16.2 | 3.2 (82) |
| | | | | | | | (67%)^{c} | (411) | (20%)^{d} |

| | | | | | | | | | |
|---|---|---|---|---|---|---|---|---|---|
| ^{a}The numbered entries refer to the wet wipes employing anti-blocking agent; ^{b}The entries with letter designators refer to comparative examples: ^{c}Percentage ratio of example sheet-to-sheet adhesion to that of Entry F; ^{d}Percentage ratio of residual soak strength at 1 h to that of the In-Use strength. | | | | | | | | | |

Table 3 provides sheet-to-sheet adhesion values for wet wipe prototypes prepared from pilot-scale nonwoven web basesheets, some of which include different anti-blocking coatings. The previously described lab-scale offset rotogravure printer with an 8 BCM gravure roll was used to apply the printing compositions of Table 3. A basesheet produced on the pilot-scale airlaid machine, described previously, was used in these examples. The airlaid basesheet employed in Table 3 comprises 80% CF405 pulp, 20% cationic ion-sensitive polyacrylate, with a total basis weight of 60 gsm and dry caliper of 1.2 mm.

Entry G illustrates an airlaid basesheet where the binder composition contains only the cationic ion-sensitive polyacrylate with a high sheet-to-sheet adhesion of 0.4 g/mm (9 g/in).

Entry H illustrates the basesheet of Entry G coated with ca. 2% (relative to the total weight of the basesheet) of FTS-226, which is a 50:50 mixture of a non-aminofunctional polyether polysiloxane and a hydrophobic aminofunctional polydimethysiloxane available from GE Silicones of Friendly, WV. Polysiloxanes are often used to reduce coefficient of friction when applied to surfaces of materials. However, the polysiloxane coating provided no beneficial impact on reducing the sheet-to-sheet adhesion versus the control basesheet G.

The Resyn® 225-A anti-blocking material (entry 8) proved to be an effective anti-blocking agent in the binder composition and also functioned to reduce the sheet-to-sheet adhesion when applied topically to the basesheet as an anti-blocking coating as indicated by the decrease of the sheet-to-sheet adhesion to 67% of the control, uncoated wet wipe.

Entry 9 in Table 3 demonstrates topical application of LATRIX® 6300 (Nalco Chemical Co. of Naperville, IL), a styrene-butadiene copolymer emulsion with a T_{g} of +55 °C. LATRIX® 6330 is an anionic emulsion material which could not be used as an antiblocking agent (i.e. part of the binder composition) as mixing of this additive with the cationic ion-sensitive polyacrylate would result in significant coagulation. However, it functions effectively as an anti-blocking coating by reducing the sheet-to-sheet adhesion to 44% of the control, uncoated wet wipe (entry G).

Entry 10 demonstrates the use of Rhoplex® ECO-4015 (Rohm and Haas, Inc. of Philadelphia, PA), which is a nonionic, acrylic emulsion with a T_{g} of +91 °C, as the antiblocking coating. This material is a particularly effective antiblocking coating as indicated by the reduction of the sheet-to-sheet adhesion to 33% of the uncoated wet wipe.

Entry 11 demonstrates the use of an anionic aqueous thermoplastic polymer dispersion, referred to herein as APD, (Dow Chemical Company of Midland, MI) as a coating. APD is an anionic aqueous polymer dispersion (solids content of 42 wt.%) based on (a) 56 wt.% of an ethylene-octene interpolymer with a T_{g} of -52 °C, Tₘ of 67 °C and a percent crystallinity of about 10%, and a density of about 0.87 g/cm3 and a melt index (ASTM D-1238, condition 190 C/2.16 kg) of about 5 g/10 min, and (b) 38 wt.% of an ethylene-acrylic-acid copolymer having about 20.5 wt. % acrylic acid and a melt index (ASTM D1238, condition 125 C/2.16 kg) of about 13-14 g/10 min, and (c) 6 wt.% of an oleic acid INDUSTRENE™ 106 (partially neutralized with KOH). This material could not be used as an anti-blocking agent with the cationic ion-sensitive polyacrylate due to the anionic charge of the APD. Despite the considerably low T_{g}, the material maintains effective sheet-to-sheet adhesion reduction to 56% of the control, uncoated wet wipe due to the high melting temperature of 67 °C.

**Table 3**

| Entry^{a,b} | Coating Material | Solution Solids (%) | Sheet-to-sheet Adhesion g/mm (g/in) |
|---|---|---|---|
| G | none, uncoated | N/A | 0.35 (9) |
| | | | (100%)^{c} |
| H | FTS-226 | 30 | 0.35 (9) |
| | | | (100%)^{c} |
| 8 | Resyn® 225-A | 30 | 0.24 (6) |
| | | | (67%)^{c} |
| 9 | Latrix 6300 | 30 | 0.16(4) |
| | | | (44%)^{c} |
| 10 | Rhoplex® ECO-4015 | 30 | 0.12 (3) |
| | | | (33%)^{c} |
| 11 | APD | 30 | 0.20 (5) |
| | | | (56%)^{c} |

| | | | |
|---|---|---|---|
| ^{a}The numbered entries refer to the wet wipes employing anti-blocking coating; ^{b}The entries with letter designators refer to comparative examples; ^{c}Percentage ratio of sheet-to-sheet adhesion to that of Entry G. | | | |

Table 4 provides sheet-to-sheet adhesion values, in-use strengths and residual soak strengths for wet wipe prototypes prepared from airlaid basesheets, some of which include anti-blocking coatings. The previously described lab-scale offset rotogravure printer was used to apply the printing compositions (antiblocking coatings) listed in Table 4 at the designated solution solids using an 12.4 MCM (8 BCM) gravure roll. The basesheet used in Table 4 was produced on a large-scale airlaid machine. The basesheet comprises 76% CF405 pulp, 24% cationic ion-sensitive polyacrylate, with a total basis weight of 63.4 gsm and dry caliper of 1.2 mm.

The control, uncoated wet wipes of entry I demonstrate a sheet-to-sheet adhesion of 203 mm (8 g/in). Entry 12 demonstrates application of the Rhoplex® ECO-4015 anti-blocking coating on this basesheet which contains a higher cationic ion-sensitive polyacrylate content in the airlaid nonwoven web. The antiblocking coating still demonstrates an effective drop in sheet-to-sheet adhesion to 63% of the uncoated wet wipe.

Vinac® 21 (Air Products Polymers, L.P. of Allentown, PA) is a poly(vinyl alcohol)-stabilized poly(vinyl acetate) latex with a T_{g} of +35 °C. The use of this material as an anti-blocking coating is demonstrated in entry 13 with a decrease in sheet-to-sheet adhesion to 63% of the uncoated wet wipe.

The APD (entry 14) coated onto the same basesheet demonstrates a drop of sheet-to-sheet adhesion to 75% of the uncoated wet wipe.

**Table 4**

| Entry^{a,b} | Anti-blocking Coating | Solution Solids (%) | Sheet-to-sheet Adhesion g/mm (g/in) | Machine Direction Wet Tensile g/mm (g/in) | | |
|---|---|---|---|---|---|---|
| | | | | In-Use MDWT | Residual MD Soak Strength in DI water, 1 h | Residual MD Soak Strength in DI water, 3 h |
| 1 | none, | N/A | 0.31 (8) | 13.8 | 1.02 (26) | 0 (0) (0%)^{d} |
| | uncoated | | (100%)^{c} | (352) | (7%)^{d} | |
| 12 | Rhoplex | 42.5 | 0.20 (5) | 12.7 | 0.91 (23) | 0 (0) (0%)^{d} |
| | ® ECO-4015 | | (63%)^{c} | (323) | (7%)^{d} | |
| 13 | Vinac® | 30.0 | 0.20 (5) | 12.1 | 0.28(7) | 0 (0) (0%)^{d} |
| | 21 | | (63%)^{c} | (307) | (2%)^{d} | |
| 14 | APD | 30.0 | 0.24 (6) | 13.1 | 1.06 (27) | 0 (0) (0%)^{d} |
| | | | (75%)^{c} | (334) | (8%)^{d} | |

| | | | | | | |
|---|---|---|---|---|---|---|
| ^{a}The numbered entries refer to the wet wipes employing anti-blocking coating; ^{b}The entries with letter designators refer to comparative examples; ^{c}Percentage ratio of example sheet-to-sheet adhesion to that of Entry I; ^{d} Percentage ratio of residual soak strength at either 1 or 3 h to that of the in-use strength. | | | | | | |

Table 5 provides sheet-to-sheet adhesion values, in-use wet strengths and residual soak strengths for wet wipe prototypes made from airlaid basesheets containing the cationic ion-sensitive polyacrylate and an anti-blocking agent in the binder composition and with anti-blocking coatings applied at varied levels using different rotogravure printer configurations. The previously described pilot-scale rotogravure printer was used for application of the anti-blocking coatings noted in Table 5 using the designated rotogravure roll, solution solids, and printer configuration. Basesheet produced on the large-scale airlaid machine was used for these examples. The basesheet comprises 79% CF405 pulp, 16.8% cationic ion-sensitive polyacrylate, 4.2% Rhoplex® ECO-4015 anti-blocking agent, with a total basis weight of 76 gsm and dry caliper of 1.3 mm

Entry 15 demonstrates the use of the Rhoplex® ECO-4015 as antiblocking agent in the binder composition where the antiblocking agent is 20 wt% of the total binder composition. Entries 16-21 demonstrate use of the RHOPLEX® ECO-4015 as anti-blocking coating applied to the basesheet of entry 15. Application of a higher amount of material with the 12.4/10.9 MCM (8/7 BCM) rotogravure rolls (approximately 2 wt% addition) and a lower amount of material with the 5/4 BCM rotogravure rolls (approximately 1 wt% addition) in either offset or direct configuration of the printer results in effective decreases of the sheet-to-sheet adhesion to the 3-4 g/in range, which is 50-67% of the uncoated wet wipe.

Entries 22-25 demonstrate use of the APD aqueous thermoplastic emulsion as anti-blocking coating on the basesheet of entry 15. Again, a decrease of the sheet-to-sheet adhesion of the wet wipe of 50-67% of the uncoated wet wipe (entry 15) is observed with sheet-to-sheet adhesion values of 3-4 g/in.

For all entries in Table 5, no impact of anti-blocking coating material or coating amount is observed on either the in-use wet strength of the wet wipe prototypes or the residual soak strengths.

**Table 5**

| Entry^{a,b} | Anti-blocking Coating | Rotogravure Roll Cell Size (BCM) | Solution Solids (%) | Configuration | Sheet-10-Sheet Adhesion g/mm (g/in) | Machine Direction Wet Tensile g/mm (g/in) | | |
|---|---|---|---|---|---|---|---|---|
| | | | | | | In-Use MDWT | Residual MD Soak Strength in DI water. 1 h | Residual MD Soak Strength in DI water. 5 h |
| 15 | none. | N/A | N/A | N/A | 0.24 (6) | 13.2 | 1.02 (26) | 0.79 (20) |
| | uncoated | | | | (100%)^{c} | (336) | (8%)^{d} | (6%)^{d} |
| 16 | Rhoplex ® ECO-4015 | 8/7 | 42.5 | offset | 012 (3) | 12.6 | 1.42 (36) | 1.50 (38) |
| | | | | | (50%)^{c} | (320) | (11%)^{d} | (12%)^{d} |
| 17 | | 8/7 | 30.0 | offset | 0.12 (3) | 13.1 | 1 34 (34) | 1.02 (26) |
| | | | | | (50%)^{c} | (333) | (10%)^{d} | (8%)^{d} |
| 18 | | 5/4 | 42.5 | offset | 0.16 (4) | 13.3 | 1.42 (36) | 1.18 (30) |
| | | | | | (67°%)^{c} | (337) | (11%)^{d} | (9%)^{d} |
| 19 | | 5'4 | 30 | offset | 0.16 (4) | 12.4 | 1.38 (35) | 1.14 (29) |
| | | | | | (67%)^{c} | (316) | (11%)^{d} | (9%)^{d} |
| 20 | | 8.7 | 42.5 | direct | 0.16 (4) | 13.3 | 146 (37) | 114 (29) |
| | | | | | (67%)^{c} | (337) | (11%)^{d} | (9%)^{c} |
| 21 | | 8/7 | 30.0 | direct | 0.12 (3) | 14.0 | 1.34 (34) | 0.98 (25) |
| | | | | | (50%)^{c} | (355) | (10%)^{d} | (7%)^{c} |
| 22 | APD | 8/7 | 40.0 | offset | 0.12 (3) | 15.0 | 1.30 (33) | 1.22 (31) |
| | | | | | (50%)^{c} | (382) | (9%)^{d} | (8%)^{d} |
| 23 | | 8/7 | 35.0 | offset | 0 12 (3) | 13.2 | 1.42 (36) | 067 (17) |
| | | | | | (30%)^{c} | (336) | (11%)^{d} | (5%)^{d} |
| 24 | | 5/4 | 35 | offset | 0 16 (4) | 13.4 | 1.18 (30) | 1.10 (28) |
| | | | | | (67%)^{c} | (341) | (9%)^{d} | (8%)^{d} |
| 25 | | 8/7 | 35 | direct | 0.16 (4) | 12.7 | 0 94 (24) | 0.98 (25) |
| | | | | | (67%)^{c} | (322) | (7%)^{a} | (8%)^{d} |

| | | | | | | | | |
|---|---|---|---|---|---|---|---|---|
| ^{a}The numbered entries refer to the wet wipes employing anti-blocking coating; The entries with letter designators refer to comparative examples; ^{c} Percentage ratio of example sheet-to-sheet adhesion to that of Entry 15; ^{d}Percentage ratio of residual soak strength at either 1 or 5 h to that of the In-Use strength. | | | | | | | | |

Table 6 provides sheet-to-sheet adhesion values, in-use wet strengths, and residual soak strengths for wet wipe prototypes prepared from basesheets incorporating different combinations of anti-blocking agents and anti-blocking coatings. The basesheets in Table 6 were coated via gravure printing using the designated gravure rolls, solution solids and printer configuration. The previously described large-scale flexographic printer was used for application of the APD coating at 38.5% solution solids. An average application of about 3% anti-blocking coating was achieved relative to the original weight of the uncoated basesheet.

Entry J is a wet wipe containing only the cationic ion-sensitive polyacrylate in the binder composition with no anti-blocking coating and thus exhibits a high sheet-to-sheet adhesion of 0.35 g/mm (9 g/in). In entry 26, flexographic printing of this basesheet with the APD anti-blocking coating reduces the sheet-tosheet adhesion of the wet wipe down to 0.16 g/mm (4 g/in) with no change in the in-use wet strength or residual soak tensile values. In entry 27, the airlaid basesheet contains a binder composition with 80% cationic ion-sensitive polyacrylate and 20% RHOPLEX® ECO-4015 anti-blocking agent, and demonstrates wet wipe prototypes with a sheet-to-sheet adhesion of 0.24 g/mm (6 g/in) (67% of entry J), which contains no RHOPLEX® ECO-4015 in the binder composition. A drop of in-use wet-strength is noted upon addition of the anti-blocking agent to the binder composition. Flexographic printing of APD onto the basesheet of entry 27 produced the wet wipe of entry 28, which demonstrates wet wipe prototypes with a further reduced sheet-to-sheet adhesion of 0.12 g/mm (3 g/in) or 33% of the sheet-to-sheet adhesion of entry J which contains no anti-blocking agents in the basesheet composition. Comparison of entries J, 26, 27, and 28 demonstrate no significant impact of the anti-blocking agent or anti-blocking coatings, or combinations thereof, on the residual soak strengths, with all entries demonstrating 1.18 g/mm (<30 g/in) of residual soak strength after 1 and 5 h. These results demonstrate that a combination of an anti-blocking agent and anti-blocking coating (entry 28) can be more effective than use of an anti-blocking coating alone (entry 26), which can be more effective than the use of an anti-blocking agent alone (entry 27) as indicated by the relative change in sheet-to-sheet adhesion values.

**Table 6**

| Entry^{a.b} | Anti-blocking Coating | Airlaid Basesheet Composition | RHOPLEX® ECO-4015 content in Binder Composition^{c} (%) | Sheet-to-sheet Adhesion g/mm (g/in) | Machine Direction Wet Tensile g/mm (g/in) | | |
|---|---|---|---|---|---|---|---|
| | | | | | In-Use MDWT | Residual MD Soak Strength in D1 water. 1h | Residual MD Soak Strength in D1 water, 5h |
| J | none. | 79% CF405 | 0 | 0.35 (9) | 14.9 | 0 (0) | 0(0) |
| | uncoated | pulp, 21% | | (100%)^{d} | (379) | (0%)^{c} | (0%)^{c} |
| | | LX7170-03: | | | | | |
| | | BW=76 gsm; | | | | | |
| | | Caliper=1.3 | | | | | |
| | | mm | | | | | |
| 26 | APD | 79% CF405 | 0 | 0.16(4) | 14.0 | 0(0) | 0(0) |
| | | pulp, 21% | | (44%)^{d} | (355) | (0%)^{e} | (0%)^{e} |
| | | LX7170-03: | | | | | |
| | | BW=76 gsm; | | | | | |
| | | Caliper= 1.3 | | | | | |
| | | mm | | | | | |
| 27 | none. | 79%CF405 | 20% | 0.24 (6) | 12.9 | 0.94(24) | 0(0) |
| | uncoated | pulp, 16.8% | | (67%)^{d} | (327) | (7%)^{c} | (0%)^{c} |
| | | LX7170-03, | | | | | |
| | | 4.2% | | | | | |
| | | Rhoplex® | | | | | |
| | | EC0-4015; | | | | | |
| | | BW=74 gsm: | | | | | |
| | | Caliper= 1.3 | | | | | |
| | | mm | | | | | |
| 28 | APD | 79%CF405 | 20% | 0.12 (3) | 13.8 | 0.71 (18) | 0.43(11) |
| | | pulp, 16.8% | | (33%)^{d} | (350) | (5%)^{e} | (3%)^{e} |
| | | 4.2% | | | | | |
| | | Rhoplex® | | | | | |
| | | EC0-4015: | | | | | |
| | | BW=74 gsm; | | | | | |
| | | Caliper=1.3 | | | | | |
| | | mm | | | | | |

| | | | | | | | |
|---|---|---|---|---|---|---|---|
| ^{a}The numbered entries refer to the wet wipes employing anti-blocking agent and/or coating; ^{b}The entries with letter designators refer to comparative examples; ^{c}The antiblocking agent content refers to the amount of anti-blocking agent included in the binder composition; ^{d}Percentage ratio of example sheet-to-sheet adhesion to that of Entry J; ^{e}Percentage ratio of residual soak strength at either 1 or 3 h to that of the In-Use strength. | | | | | | | |

The previous tables demonstrated the properties of wet wipe prototypes that were hand-converted and artificially aged to represent actual packaged wet wipe product sheet-to-sheet adhesion characteristics. Table 7 demonstrates a comparison of the properties of machine-converted dispersible packaged wet-wipes to that of other dispersible and non-dispersible packaged wet wipes, the majority of which are commercially available.

In-use wet tensile strengths for the packaged wet wipes in Table 7 were measured in both the MD and CD directions. For the folded wet wipes, the CD direction is the dispensing direction of the wet wipe. For the wet wipes in the rolled format, the dispensing direction is the MD direction. Packaged wet wipe sheet-to-sheet adhesion was measured in the MD direction between wipes in both the folded and rolled formats. Wet opacity and wet sheet thickness of the packaged wet wipe products were evaluated as described in the *General. Procedures* section. The dispersibility of the packaged wet wipes was evaluated in both a "soft water" and "tap water" simulant using static soak and slosh box test methods. The solution referred to as "soft water" for both the soak test and slosh box test, has a total dissolved solids of ca. 31.5 ppm and a CaCO₃ equiv water hardness of ca. 30 ppm, would be considered "soft water" as defined by the USGS. This solution represents the lower end of the water composition range that would be typical of tap water. The solution for the "tap water" for both the soak test and slosh box test has a total dissolved solids of 500 ppm and a calculated water hardness of about 248 ppm equivalents CaCO₃ This "tap water" simulant would be classified as "very hard" as defined by the USGS. As discussed previously, this "tap water" solution should adequately encompass the vast majority of tap water compositions present in households across the United States given the municipal water composition data available.

Entry 29 demonstrates packaged wet-wipes derived from the same airlaid basesheet described for Entry 28. The basesheet was machine-converted into sections of continuous web 140 mm (5.5") wide by 1.42 m (56") long with perforations every 178 mm (7") which were adhesively joined, fan-folded and applied with the wetting composition at 250% add-on to yield a fan-folded stack of wet-wipes. The fan-folded stacks contained 42 140 mm × 178 mm (5.5"×7") wet wipes which were packaged into shrinkwrapped plastic tubs. An aqueous wetting composition that is used on commercially available wet wipes under the trade designation KLEENEX® COTTONELLE FRESH® Folded Wipes (Kimberly-Clark Corporation of Neenah, WI) with the addition of 2 wt% sodium chloride was applied in the wet-wipe converting process. The packaged wet wipe demonstrates an acceptable In-Use MDWT strength with a variance of ca. 5% with wet-tensile strength loss significantly out of that range upon soaking in either the tap water or soft water solutions, with faster tensile loss observed in the soft water solution versus the tap water solution. Dynamic break-up in the slosh-box to pieces of less than 25.4 mm (1") square area occurred in ca. 95 min in the tap water solution and only ca. 32 min in the soft water solution. The sheet-to-sheet adhesion measured from this flat, fan-folded packaged wet wipe is 0.08 g/mm (2 g/in) which is comparable to a similar non-dispersible adhesively-bonded wet wipe of Entry L. The product demonstrated acceptable dispensing performance.

Entry 30 demonstrates packaged wet wipes derived from the airlaid basesheet of entry 26 and were machine converted into fan-folded wet wipes in the process described for Entry 29. The packaged wet wipes of Entry 30 demonstrate higher In-Use Strength than that of Entry 29 due to the absence of the anti-blocking agent in the basesheet, which does not contribute effectively to the wet-strength of the binder composition. The packaged wet wipe demonstrates an in-use MDWT tensile variance of ca. 5% and demonstrated wet-tensile strength loss significantly out of that range upon statically soaking in either the tap water or soft water solutions, with faster tensile loss observed in the soft water solution versus the tap water solution. Dynamic break-up in the slosh-box to pieces of less than 645 mm² (1" square) area occurred in ca. 90 min in the tap water solution and only ca. 40 min in the soft water solution. The sheet-to-sheet adhesion measured from this flat, fan-folded packaged wet wipe is 0.12 g/mm (3 g/in) which is slightly higher than the other folded wet wipes in Table 7, but is still sufficiently low to provide acceptable dispensing.

Entry K demonstrates packaged wet wipes derived from an adhesively-bonded airlaid basesheet with a composition of 83% CF405 pulp; 12.75% an anionic ion-sensitive polyacrylate of the composition 60% acrylic acid, 24.5% n-butyl acrylate, 10.5% 2-ethylhexyl acrylate, and 5% acrylamide-2-propane sulfonic acid; 4.25% DUR-O-SET® RB (National Starch and Chemical Co. of New Brunswick, NJ); a basis weight of 60 gsm; and a dry caliper of 1.0 mm. The basesheet was slit into 102 mm (4") wide coreless rolls with perforations every 109 mm (4-3") to yield 100 102 mm × 109 mm (4.0" x4.3") sized wet wipes per roll. An aqueous wetting composition containing 4% NaCl along with small amounts of preservatives, surfactants, dimethiconol, and fragrance was applied to the rolls at a 225% solution add-on. The coreless wet wipe rolls were packaged in a sealed plastic cartridge. The product of entry K demonstrates an in-use tensile variance of about 5% and significant tensile loss is observed out of that range when statically soaked in either tap water or soft water solutions. Dynamic break-up in the slosh-box to pieces of less than 645 mm² (1" square) area does not occur within 24 h in the tap water solution but does occur quite rapidly the soft water solution. The sheet-to-sheet adhesion of this rolled wet wipe product is 0.43 g/mm (11 g/in) which is very high compared to the other entries in the table. The high sheet-to-sheet adhesion makes the wet wipes considerably difficult to dispense from the roll.

Entry L is COTTONELLE FRESH® Folded Wipes which is a flushable premoistened personal cleansing wipe distributed by Kimberly-Clark Corporation of Neenah, WI. The substrate of the product is an adhesively-bonded airlaid basesheet which is dispensed in a flat, z-folded stack of individual wet-wipes (reach-in format). The product demonstrates an in-use tensile variance of about 5% and no significant tensile loss is observed out of that range when statically soaked in tap water soft water solutions. Dynamic break-up in the slosh-box to pieces less than 645 mm² (1" square in) area does not occur within 24 h in the tap water or soft water solutions. The sheet-to-sheet adhesion of this packaged non-dispersible wet wipe product was very low, around 0.08 g/mm (2 g/in).

Entry M is Charmin® Fresh Mates, a flushable, premoistened personal cleansing wipe distributed by Proctor & Gamble of Cincinnati, OH. The substrate of the product is a hydroentangled basesheet which is dispensed in a flat, folded reach-in format. No tensile strength loss was observed outside of the in-use tensile strength variance of about 28% in the tap water or soft water solutions. Dynamic break-up in the slosh-box to pieces less than 645 mm² (1" square in) area does not occur within 24 h in the tap water or soft water solutions. The sheet-to-sheet adhesion of this packaged non-dispersible wet wipe product was very low, about 0.04 g/mm (1 g/in).

Entry N is KLEENEX® Fresh Bidet Wipes, a flushable premoistened personal cleansing wipe distributed by Yuhan-Kimberly in Korea. The substrate of the product is a hydroentangled basesheet which is dispensed in a flat, interfolded format. No tensile strength loss was observed outside of the in-use tensile strength variance of about 23% when statically soaked in the tap water or soft water solutions. Dynamic break-up in the slosh-box to less than 645 mm² (1" square) pieces occurs in the tap water and soft water solutions after about 6 to 14 hours. The sheet-to-sheet adhesion of this packaged wet wipe product was very low, around 0.04 g/mm (1 g/in).

Entry O is Scrubbing Bubbles® Flushable Bathroom Wipes, a premoistened wipe for bathroom fixture cleaning distributed by S.C. Johnson & Son, Inc. The substrate of the product is a hydroentangled basesheet which is dispensed in a flat, folded reach-in format. No tensile strength loss was observed outside of the in-use tensile strength variance of about 16% when statically soaked in the tap water or soft water solutions. Dynamic break-up in the slosh- box to pieces less than 645 mm² (1"square in) area occurs in the tap water and soft water solutions after about 4 to 6 hours. The sheet-to-sheet adhesion of this packaged wet-wipe product was very low, around 0.04 g/mm (1 g/in).

**Table 7**

| Entry^{a.b} | In-Use MDWT g/mm (g/in) | In-Use CDWT g/mm (g/in) | Tap water, residual MD soak tensile g/mm (g/in) | Slosh box breakup in Tap water simulant (min) | Soft water, residual MD soak tensile g/mm (g/in) | slosh box breakup in soft water (min) | Sheet-to-sheet adhesion g/mm (g/in) | Wet- sheet thickness (mm) | Opacity (%) |
|---|---|---|---|---|---|---|---|---|---|
| | | | 3.9 (99) (1 h) [27%]^{c} | | 2.01 (51) (1 h) [14%] | | | | |
| 29 | 14.6 (372) | 11.8 (300) | 2.3 (59) (3h) [16%]^{c} | 94 | 1.46 (37) (3h) [10%] | 32 | 0.08 (2) | 0.56 | 47 |
| | | | 2.2 (57) (5h) [15%]^{c} | | 0.98 (25) (5 h) [7%] | | | | |
| | | | 3.3 (83) (1h) [18%]^{c} | | 1.38 (35) (1 h) [7%] | | | | |
| 30 | 18.1 (461) | 14.3 (364) | 2.0 (51) (3h) [11%]^{c} | 89 | 0.75 (19) (3h) [4%] | 39 | 0.12 (3) | 0.55 | 46 |
| | | | 1.6 (41) (5h) [9%]^{c} | | 1.02 (26) (5h) [6%] | | | | |
| | | | 1,4 (36) (1h) [6%]^{c} | | 1.38 (35) (1h) [6%] | | | | |
| K | 23.5 (596) | 16.8 (427) | 1.3 (34) (3 h) [6%]^{c} | > 1440 | 1.42 (36) (3 h) [6%] | 14 | 0.43 (11) | 0.42 | 44 |
| | | | 1.3(31) (5h) [6%]^{c} | | 1.38 (35) (5h) [6%] | | | | |
| | | | 14.4 (366) (1h) [91%] | | 14.1 (358) (1h) [89%] | | | | |
| L | 15.8 (402) | 11.6 (295) | 14,8 (376) (3h) [94%] | >1440 | 17.3 (439) (3h) [109%] | >1440 | 0.08 (2) | 0.60 | 50 |
| | | | 16.8 (426) (5 h) [106%] | | 15.7 (398) (5 h) [99%] | | | | |
| | | | 159.1 (4041) (1 h) [84%]^{c} | | 171.1 (4.345) (1 h) [91%] | | | | |
| M | 188.9 (4799) | 33.1 (840) | 185.6 (4714) (3 h) [98%]^{c} | >1440 | 165.5 (4204) (3 h) [88%] | >1440 | 0.4 (1) | 0.50 | 52 |
| | | | 143.8 (3653) (5 h) [76%]^{c} | | 173.0 (4394) (5 h) [92%] | | | | |
| | | | 11.1 (282) (1 h) [87%]^{c} | | 14.1 (358) (1 h) [110%] | | | | |
| N | 12.8 320) | 11.6 (294) | 10,8 (275) (3 h) [84%]^{c} | 537 | 15.1 (383) (3 h) [117%] | 840 | 0.04 (1) | 0.47 | 48 |
| | | | 12.9 (327) (5 h) [100%]^{c} | | 15.9 (404) (5 h) [124%] | | | | |
| | | | 15.1 (383) (1 h) [96%]^{c} | | 10.1 (256) (1 h) [64%] | | | | |
| O | 15.7 (399) | 5.3 (135) | 11. 7 (297) (3 h) [74%] | 345 | 9.9 (252) (3 h) [63%] | 254 | 0.04 (1) | 0.47 | 45 |
| | | | 15.1 (384) (5 h) [96%] | | 11.1 (282) (5 h) [71%] | | | | |

| | | | | | | | | | |
|---|---|---|---|---|---|---|---|---|---|
| ^{a}The numbered entries refer to the wet wipes employing anti-blocking agent and/or coating; ^{b}The entries with letter designators refer to comparative examples; ^{c}Percentage ratio of residual soak strength at either 1, 3 or 5 h to that of the In-Use strength. | | | | | | | | | |

Table 8 demonstrates reduced sheet-to-sheet adhesion of artificially aged dry, adhesively-bonded airlaid basesheets upon application of an anti-blocking coating to the airlaid basesheet, in the absence of an anti-blocking agent. Entry P provides dry sheet-to-sheet adhesion for the dry airlaid basesheet of entry 1, which contains no anti-blocking agent or anti-blocking coating in the basesheet. Entry 31 and Entry 32 demonstrate dry sheet-to-sheet adhesion values for the dry airlaid basesheets described in Entries 12 and 14, respectively, which contain anti-blocking coatings. Use of an anti-blocking coating results in a drop in the sheet-to-sheet adhesion to 18% of the uncoated basesheet with the Rhoplex® ECO-4015 and a drop to 36% of the uncoated basesheet with the APD.

**Table 8**

| Entry^{a, b} | Anti-blocking Coating | Dry sheet-to-sheet adhesion g/mm (g/in) |
|---|---|---|
| P | none (control) | 0.43 (11) (100%) |
| 31 | Rhoplex® ECO-4015 | 0.08 (2) (18%) |
| 32 | APD | 0.16 (4) (36%) |

| | | |
|---|---|---|
| ^{a}The numbered entries refer to the wet wipes employing anti-blocking coating; ^{b}The entries with letter designators refer to comparative examples; | | |

## Claims

1. A wet wipe comprising:
a nonwoven material comprising:
a fibrous material;
a binder composition comprising a triggerable polymer;
an anti-blocking agent having a glass transition temperature or a melting temperature of about 23 °C or higher; wherein the anti-blocking agent is present as a component of the binder composition or is present as an anti-blocking coating on the surface of the fibrous material; and
an aqueous-wetting composition, wherein the binder composition is insoluble in the wetting composition,
wherein the wet wipe is dispersible in water having a total dissolved solids up to about 500 ppm and a CaCO₃ equivalent hardness up to about 250 ppm.

2. The wet wipe of claim 1, wherein the binder composition further comprises a cobinder.

3. The wet wipe of any of claims 1 or 2, wherein the anti-blocking agent is present as an anti-blocking coating on the surface of the fibrous material, the binder composition further comprising an anti-blocking agent as a component of the binder composition.

4. The wet wipe of any of the preceding claims, wherein the anti-blocking agent is a polymer selected from a vinyl acetate-ethylene copolymer emulsion, an acrylic emulsion, and a poly(vinyl acetate) emulsion.

5. The wet wipe of any of the preceding claims, wherein the anti-blocking coating is an aqueous thermoplastic polyolefin dispersion.

6. The wet wipe of any of the preceding claims, wherein the sheet-to-sheet adhesion is less than about 2,36 g/cm (6 g/in).

7. The wet wipe of claim 6, wherein the sheet-to-sheet adhesion is less than about 1,18 g/cm (3 g/in).

8. The wet wipe of any of the preceding claims, wherein the triggerable polymer is the polymerization product of methyl acrylate and [2-(acryloxy)ethyl] trimethyl ammonium chloride.

9. The wet wipe of any of the preceding claims, which has a tensile strength of less than about 39,37 g/cm (100 g/in) after being soaked for about one hour in water having a total dissolved solids up to 500 ppm and a CaCO3 equivalent hardness up to about 250 ppm.

10. The wet wipe of any of the preceding claims, which has a slosh box break-up time less than about 300 minutes in water having a total dissolved solids up to 500 ppm and a CaCO3 equivalent hardness up to about 250 ppm.

11. The wet wipe of any of the preceding claims, wherein the content of anti-blocking agent in the binder composition is from about 5 weight % to about 40 weight % of the mass of the total binder composition.

12. The wet wipe of any of claims 1-10, wherein the content of anti-blocking coating on the wet wipe is from about 2 wt% to about 10 wt% relative to the mass of the nonwoven web.

13. The wet wipe of any of the preceding claims, wherein the nonwoven material is a nonwoven fabric.

14. The wet wipe of any of claims 1 to 12, wherein the nonwoven material is a nonwoven web.

## Patentansprüche

1. Ein Feuchttuch, umfassend:
ein Vliesmaterial, welches umfasst:
ein faseriges Material;
eine Bindezusammensetzung, welche ein auslösbares Polymer umfasst;
ein Antiblockmittel mit einer Glasübergangstemperatur oder einer Schmelztemperatur von ungefähr 23°C oder höher; wobei das Antiblockmittel als eine Komponente der Bindezusammensetzung vorhanden ist oder als Antiblockbeschichtung auf der Oberfläche des faserigen Materials vorhanden ist; und
eine wässrige Netzzusammensetzung, wobei die Bindezusammensetzung in der Netzzusammensetzung unlöslich ist,
wobei das Feuchttuch in Wasser dispergierbar ist, welches eine Gesamtmenge von gelösten Feststoffen von bis zu ungefähr 500 ppm und eine CaCO₃-Äquivalenthärt von bis zu 250 ppm aufweist.

2. Feuchttuch gemäß Anspruch 1, wobei die Bindezusammensetzung des Weiteren ein Cobindemittel umfasst.

3. Feuchttuch gemäß Anspruch 1 oder 2, wobei das Antiblockmittel als Antiblockbeschichtung auf der Oberfläche des faserigen Materials vorhanden ist, wobei die Bindezusammensetzung des Weiteren ein Antiblockmittel als eine Komponente der Bindezusammensetzung umfasst.

4. Feuchttuch gemäß einem der vorherigen Ansprüche, wobei das Antiblockmittel ein Polymer ist, welches ausgewählt ist aus einer Vinylacetat-EthylenCopolymer-Emulsion, einer Acryl-Emulsion und einer Poly(Vinylacetat)Emulsion.

5. Feuchttuch gemäß einem der vorherigen Ansprüche, wobei die Antiblockbeschichtung eine wässrige thermoplastische Polyolefin-Dispersion ist.

6. Feuchttuch gemäß einem der vorherigen Ansprüche, wobei die Schicht-an-Schicht-Haftung weniger als ungefähr 2,36 g/cm (6 g/in) beträgt.

7. Feuchttuch gemäß Anspruch 6, wobei die Schicht-an-Schicht-Haftung weniger als ungefähr 1,18 g/cm (3 g/in) beträgt.

8. Feuchttuch gemäß einem der vorherigen Ansprüche, wobei das auslösbare Polymer das Polymerisationsprodukt aus Methylacrylat und [2-(Acryloxy)Ethyl]-Trimethyl-Ammoniumchlorid ist.

9. Feuchttuch gemäß einem der vorherigen Ansprüche, welches eine Zugfestigkeit von weniger als ungefähr 39,37 g/cm (100 g/in) nach dem Tränken für eine Stunde in Wasser aufweist, welches eine Gesamtmenge an gelösten Feststoffen von bis zu 500 ppm und eine CaCO₃-Äquivalenthärte von bis zu 250 ppm aufweist.

10. Feuchttuch gemäß einem der vorherigen Ansprüche, welches eine Slosh-Box Zerfallszeit von weniger als ungefähr 300 Minuten in Wasser aufweist, welches eine Gesamtmenge an gelösten Feststoffen von bis zu 500 ppm und eine CaCO₃ Äquivalenthärte von bis zu 250 ppm aufweist.

11. Feuchttuch gemäß einem der vorherigen Ansprüche, wobei der Gehalt an Antiblockmittel in der Bindezusammensetzung von ungefähr 5 Gewichtsprozent bis ungefähr 40 Gewichtsprozent der Masse der GesamtBindezusammensetzung beträgt.

12. Feuchttuch gemäß einem der Ansprüche 1-10, wobei der Gehalt an Antiblockbeschichtung auf dem Feuchttuch von ungefähr 2 Gew.-% bis ungefähr 10 Gew.-% der Masse der Vliesbahn beträgt.

13. Feuchttuch gemäß einem der vorherigen Ansprüche, wobei das Vliesmaterial ein Vliesstoff ist.

14. Feuchttuch gemäß einem der Ansprüche 1 bis 12, wobei das Vliesmaterial eine Vliesbahn ist.

## Revendications

1. Lingette humide comprenant :
un matériau non tissé comprenant :
un matériau fibreux ;
une composition de liant comprenant un polymère activable ;
un agent antibloquant ayant une température de transition vitreuse ou une température de fusion d'environ 23 °C ou supérieure ; dans laquelle l'agent antibloquant est présent en tant que composant de la composition de liant ou est présent sous la forme d'un revêtement antibloquant sur la surface du matériau fibreux ; et
une composition humidifiante aqueuse, dans laquelle la composition de liant est insoluble dans la composition humidifiante,
dans laquelle la lingette humide est dispersible dans de l'eau ayant une teneur totale en matières solides dissoutes jusqu'à environ 500 ppm et une dureté équivalente en CaCO₃ jusqu'à environ 250 ppm.

2. Lingette humide selon la revendication 1, dans laquelle la composition de liant comprend en outre un co-liant.

3. Lingette humide selon l'une quelconque des revendication 1 ou 2, dans laquelle l'agent antibloquant est présent sous la forme d'un revêtement antibloquant sur la surface du matériau fibreux, la composition de liant comprenant en outre un agent antibloquant en tant que composant de la composition de liant.

4. Lingette humide selon l'une quelconque des revendication précédentes, dans laquelle l'agent antibloquant est un polymère choisi parmi une émulsion de copolymère d'éthylène-acétate de vinyle, une émulsion acrylique et une émulsion de poly(acétate de vinyle).

5. Lingette humide selon l'une quelconque des revendications précédentes, dans laquelle le revêtement antibloquant est une dispersion de polyoléfine thermoplastique aqueuse.

6. Lingette humide selon l'une quelconque des revendications précédentes, dans laquelle l'adhérence feuille à feuille est inférieure à environ 2,36 g/cm (6 g/in).

7. Lingette humide selon la revendication 6, dans laquelle l'adhérence feuille à feuille est inférieure à environ 1,18 g/cm (3 g/in).

8. Lingette humide selon l'une quelconque des revendications précédentes, dans laquelle le polymère activable est le produit de polymérisation de l'acrylate de méthyle et du chlorure de [2-(acryloxy)éthyl]triméthyl ammonium.

9. Lingette humide selon l'une quelconque des revendications précédentes, qui a une résistance à la traction inférieure à environ 39,37 g/cm (100 g/in) après avoir été trempée pendant environ une heure dans de l'eau ayant une teneur totale en matières solides dissoutes jusqu'à 500 ppm et une dureté équivalente en CaCO₃ jusqu'à environ 250 ppm.

10. Lingette humide selon l'une quelconque des revendications précédentes, qui a un temps de fragmentation en caisson ballottant inférieur à environ 300 minutes dans l'eau ayant une teneur totale en matières solides dissoutes jusqu'à 500 ppm et une dureté équivalente en CaCO₃ jusqu'à environ 250 ppm.

11. Lingette humide selon l'une quelconque des revendications précédentes, dans laquelle la teneur en agent antibloquant dans la composition de liant est d'environ 5 % en poids à environ 40 % en poids de la masse de la composition de liant totale.

12. Lingette humide selon l'une quelconque des revendications 1 à 10, dans laquelle la teneur en revêtement antibloquant sur la lingette humide est d'environ 2 % en poids à environ 10 % en poids rapporté à la masse du voile non tissé.

13. Lingette humide selon l'une quelconque des revendications précédentes, dans laquelle le matériau non tissé est une structure non tissée.

14. Lingette humide selon l'une quelconque des revendications 1 à 12, dans laquelle le matériau non tissé est un voile non tissé.
